# FASCICULE DE BREVET EUROPEEN

(11) **EP 0 552 357 B1**
(45) Date de publication et mention de la délivrance du brevet: **17.12.2003**
(21) Numéro de dépôt: 92918047.9
(22) Date de dépôt: 07.08.1992
(51) Int. Cl.: A61N 1/36

(54) **PROCEDE ET DISPOSITIF DE CONTROLE DE L'ENERGIE DE STIMULATION DANS UN STIMULATEUR CARDIAQUE**
VERFAHREN UND GERÄT ZUR ENERGIEREGELUNG BEIM STIMULIEREN IN EINEM HERZSCHRITTMACHER
METHOD AND DEVICE FOR CHECKING STIMULATION POWER IN A PACEMAKER

(30) Priorité: 09.08.1991 FR 9110169
(43) Date de publication de la demande: 28.07.1993
(73) Titulaire: ELA MEDICAL, F-92541 Montrouge (FR)
(72) Inventeur: DUBREUIL, Anne, F-92100 Boulogne (FR); NITZSCHE, Rémi, F-78650 Beynes (FR); WANDERSTOK, Georges, F-92140 Clamart (FR)
(74) Mandataire: Laget, Jean-Loup
(86) Numéro de dépôt international: FR9200779
(87) Numéro de publication internationale: WO93002741

(56) Documents cités:
- EP-A- 0 129 503
- EP-A- 0 334 681
- EP-A- 0 399 063

## Description

L'invention concerne un procédé de contrôle de l'énergie de stimulation dans un stimulateur cardiaque et un dispositif correspondant.

Dans un stimulateur cardiaque, l'énergie de stimulation, ventriculaire par exemple, est appliquée à la paroi du ventricule au moyen d'une électrode de stimulation sous la forme d'une impulsion dont l'amplitude est représentative d'une tension, et dont la largeur est représentative d'une durée.

L'énergie de stimulation est ainsi fonction d'une tension et d'une durée. Comme cette énergie est stockée dans une pile implantée avec le stimulateur, la durée de vie de la pile est directement liée à l'énergie de stimulation.

Une réduction de l'énergie de stimulation permet de prévoir une réduction de la taille et/ou une augmentation de la durée de vie de la pile.

Une telle réduction de l'énergie de stimulation ne peut cependant pas être envisagée sans précautions car la stimulation doit être efficace. Pour vérifier qu'une stimulation cardiaque est efficace on analyse le signal cardiaque après la stimulation, et on cherche à l'analyser le plus tôt possible après la stimulation pour pouvoir faire une contre-stimulation dans le cas où la stimulation a été inefficace. Pour être le plus près possible de la stimulation, on analyse l'onde R, dont l'amplitude est de l'ordre de 10mV. Comme le signal de stimulation a une amplitude de quelques volts, il est assez difficile de percevoir l'onde R qui est masquée par des charges électriques rémanentes au niveau de l'interface coeur-électrode. Pour déterminer si une stimulation a été efficace ou non, il faut pouvoir s'affranchir de l'influence de ces charges électriques.

Par ailleurs, l'énergie minimale de stimulation entraînant une réponse cardiaque est définie comme correspondant au seuil de capture. Pour la détermination du seuil de capture, diverses solutions ont été proposées.

Le brevet EP 0 334 681 décrit un stimulateur à vérification de capture lorsqu'une perte de capture a été détectée. La capture est vérifiée par détection d'une réponse à une impulsion de stimulation. Le fait de ne pas recueillir une réponse dans un délai de 60ms est apprécié comme une perte de capture. Dans ce cas, le stimulateur envoie une impulsion supplémentaire.

Le brevet US 3 920 024 décrit un test de seuil comportant une série de stimulations d'énergie décroissante avec analyse du signal endocavitaire entre 20 et 40 ms après la stimulation. Lors d'une stimulation inefficace, des contre-stimulations successives, espacées de 50 ms, et d'amplitude et de largeur croissantes, sont effectuées jusqu'à obtention d'une réponse cardiaque. Après un test de seuil, l'énergie de stimulation est recalculée à partir de l'énergie de la première contre-stimulation efficace. Dans ce brevet, la stimulation est assurée par l'électrode distale et la détection par l'électrode proximale. Il y a donc un changement de pôle entre la stimulation et la détection.

Le brevet EP 0 372 698 décrit un stimulateur comportant un amplificateur à capacités commutées, avec changement sélectif de la bande passante et du gain entre la détection des complexes spontanés et la vérification de la capture. Le gain est augmenté et la fréquence de coupure basse diminuée pour vérifier la capture, par exemple par détection de l'onde T. La détection de l'onde T est tardive et n'autorise pas l'émission d'une contre-stimulation.

Le but de la présente invention est de proposer un procédé de mesure du seuil de capture afin d'en déduire l'énergie de stimulation à fournir, tout en évitant les inconvénients des solutions connues.

Un autre but de l'invention est de proposer un procédé de mesure du seuil de capture qui distingue la situation chronique de la situation aiguë correspondant à une période d'environ un mois à compter de l'implantation.

Un autre but de l'invention est de suivre l'évolution du seuil de capture dans le temps pour y adapter automatiquement et périodiquement l'énergie de stimulation.

Dans un procédé de contrôle au moyen d'un logiciel, par la mesure d'un paramètre de capture, de l'énergie de stimulation dans un stimulateur cardiaque selon l'invention:
- on définit un paramètre de capture variant en fonction de l'énergie de stimulation ;
- au cours d'une phase d'étalonnage, on détermine les caractéristiques représentatives du paramètre de capture en fonction de l'énergie de stimulation ;
- à partir de ces caractéristiques, on définit une valeur de référence pour déterminer l'efficacité des stimulations ;
- au cours d'une phase de recherche de seuil, on détermine la valeur du seuil de capture à partir de la valeur de référence ;
- et on détermine l'énergie de stimulation en fonction de la valeur du seuil de capture.

Selon d'autres particularités de l'invention :
- le paramètre de capture est l'amplitude négative maximale du signal cardiaque obtenu après la stimulation, dans un délai déterminé ;
- le délai déterminé commence après le blanking et se termine environ 64 ms après la stimulation ;
- dans le cas où le paramètre de capture varie linéairement en fonction de l'énergie de stimulation, les caractéristiques de la droite représentative du paramètre de capture sont la pente (a) et l'ordonnée à l'origine (b) ;
- la valeur de référence pour déterminer l'efficacité des stimulations est une fraction de l'ordonnée à l'origine (b) égale de préférence à 3/4 ;
- au commencement de la phase d'étalonnage ou de la phase de recherche de seuil, on diminue, si cela est possible, la période du cycle cardiaque de façon à éviter le phénomène de fusion ;
- pour déterminer la valeur du seuil de capture, des séries successives d'impulsions de stimulation sont produites en faisant décroître l'amplitude des impulsions entre les séries ;
- chaque série d'impulsions est constituée de préférence de quatre impulsions de même amplitude ;
- après chaque série d'impulsions toutes efficaces, on fait décroître l'amplitude d'une quantité déterminée, de préférence 0,25 V ;
- lorsque l'amplitude des impulsions est de l'ordre de 1,25V, on garde cette valeur comme valeur de seuil ;
- après une série d'impulsions toutes inefficaces, on définit comme valeur de seuil l'amplitude des impulsions de la série précédente ;
- si le stimulateur fonctionne en mode VVI, après une série d'impulsions de stimulation ne donnant pas toutes le même résultat, on vérifie si la période du cycle cardiaque est supérieure d'au moins une durée d'environ 32ms à la période minimale autorisée : dans ce cas, on réduit de ladite durée, l'intervalle d'échappement ventriculaire et on procède à une nouvelle série d'impulsions avec la même amplitude, et dans le cas contraire on définit comme valeur de seuil l'amplitude des impulsions de la série précédente ;
- si le stimulateur fonctionne en mode DDD, après une série d'impulsions ne donnant pas toutes le même résultat, on vérifie si le délai AV est supérieur d'au moins une durée d'environ 32ms à une valeur de préférence égale à 62ms : dans ce cas, on réduit le délai AV de ladite durée, et on procède à une nouvelle série d'impulsions avec la même amplitude, et dans le cas contraire on définit comme valeur de seuil l'amplitude des impulsions de la série précédente ;
- l'énergie de stimulation est déterminée en prenant pour amplitude des impulsions de stimulation le double de la valeur du seuil de capture ;
- pendant la phase d'étalonnage, après chaque stimulation à 2,5 V, on déclenche une contre stimulation ;
- pendant la phase de recherche de seuil, en cas de stimulation inefficace, on déclenche une contre-stimulation;
- en dehors des phases d'étalonnage et de recherche de seuil, sauf en cas de stimulation à haute énergie, on vérifie l'efficacité de chaque stimulation, et en cas de stimulation inefficace, les stimulations des cycles suivants, de préférence au nombre de quatre, sont délivrées à haute énergie ;
- on évalue le taux d'efficacité des stimulations au moyen d'un compteur-décompteur auquel on applique un incrément de 1, de préférence, pour chaque stimulation efficace, et un décrément de 4, de préférence, pour chaque stimulation inefficace, et lorsque le compteur descend à 0, on définit le taux d'efficacité des stimulations comme insuffisant, et on déclenche une phase d'étalonnage ;
- pendant la phase d'étalonnage on vérifie de préférence au maximum trois fois, la cohérence des séries de stimulations ;
- pendant la phase de recherche de seuil, on procède de préférence au maximum trois fois à la réduction de la période cardiaque pour la détermination de l'efficacité des stimulations ;
- on distingue une situation chronique, et une situation aiguë correspondant à une période d'environ un mois à compter de l'implantation du stimulateur ;
- la périodicité des phases d'étalonnage est de 24 heures en situation chronique et de 6 heures en situation aiguë ;
- la périodicité des phases de recherche de seuil est de 6 heures en situation chronique et de 1 heure 30 en situation aiguë.
- lorsque la phase d'étalonnage définit pour le seuil une valeur supérieure à 2,5V, la phase de recherche de seuil n'est pas déclenchée.

L'invention a également pour objet un dispositif de contrôle de l'énergie de stimulation dans un stimulateur cardiaque comportant :
- un générateur d'impulsions pour produire des impulsions ayant une énergie de stimulation déterminée,
- un moniteur pour détecter la réponse cardiaque à l'énergie de stimulation d'une impulsion fournie,
- des moyens de commande de l'énergie de stimulation fournie par rapport à une valeur déterminée de seuil de capture,
caractérisé en ce qu'il comporte des moyens pour définir le seuil de capture selon une procédure d'étalonnage dans laquelle des caractéristiques représentatives d'un paramètre de capture constitué par l'amplitude négative maximale du signal cardiaque obtenu après la stimulation dans un délai déterminé, sont déterminées en fonction de l'énergie de stimulation analysée en réponse à une ou plusieurs impulsions, le paramètre de capture variant en fonction de l'énergie de stimulation, et dans laquelle une valeur de référence est définie pour déterminer l'efficacité d'une impulsion en réponse aux caractéristiques représentatives déterminées du paramètre de capture, et selon une procédure de recherche de seuil dans laquelle la valeur du seuil de capture est déterminée sur la base de la valeur de référence définie.

D'autres caractéristiques ressortent de la description qui suit faite avec référence aux dessins annexés sur lesquels on peut voir
Fig. 1 : un schéma simplifié de l'étage de sortie du stimulateur cardiaque selon l'invention ;
Fig. 2 : un schéma simplifié d'une chaîne de mesure du signal endocavitaire, dans le stimulateur cardiaque selon l'invention ;
Fig. 3 : un diagramme d'état de l'algorithme de mesure du seuil de capture dans le stimulateur cardiaque selon l'invention ;
Fig. 4 : un graphe représentatif du résultat de l'étalonnage, pour la mesure du paramètre de capture ;
Fig. 5 : un diagramme du logiciel d'étalonnage pour la mesure du seuil de capture dans le cas d'un stimulateur fonctionnant en mode VVI ;
Fig. 6 : un diagramme du logiciel d'étalonnage pour la mesure du seuil de capture dans le cas d'un stimulateur fonctionnant en mode DDD ;
Fig. 7 : un diagramme du logiciel de recherche du seuil de capture dans le cas d'un stimulateur en mode VVI ;
Fig. 8 : un diagramme du logiciel de recherche du seuil de capture dans le cas d'un stimulateur fonctionnant en mode DDD.

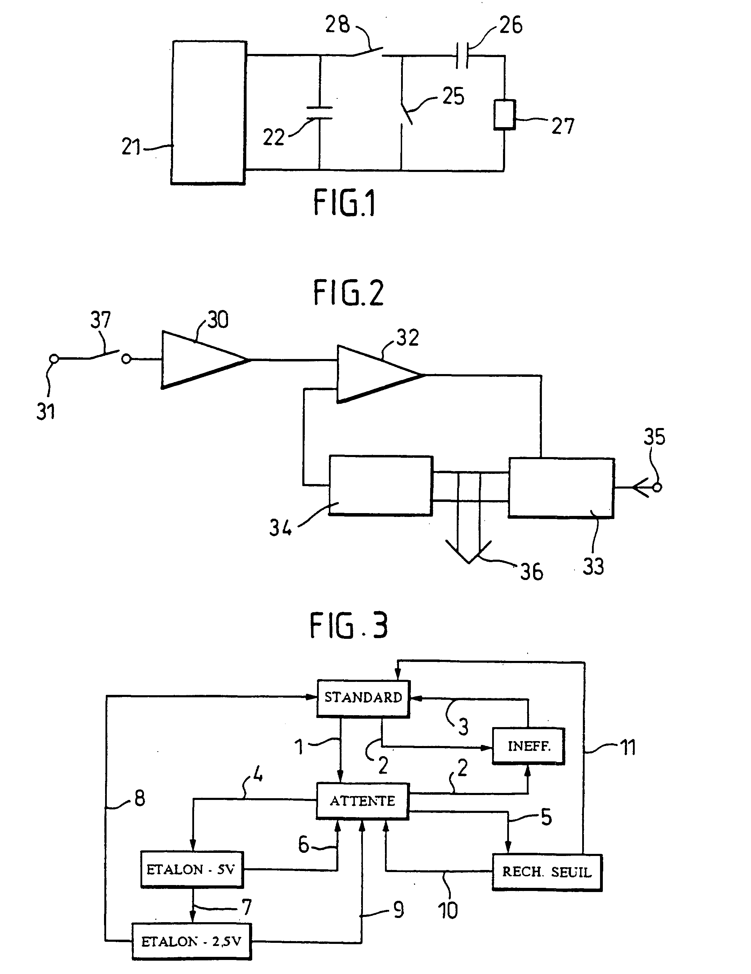

En se reportant à la Fig. 1, on voit que l'étage de sortie du stimulateur comprend essentiellement un circuit de charge 21 alimentant la capacité de stimulation 22.

Un interrupteur 25 est susceptible d'assurer la décharge de la capacité de sortie 26 et de l'interface électrode-coeur 27. Entre la capacité de charge 22 et le reste du circuit est disposé un interrupteur de stimulation 28.

Lorsque la capacité 22 est chargée, l'interrupteur de stimulation 28 est fermé pendant la stimulation, et la capacité de stimulation 22 se décharge partiellement dans la capacité 26 et dans l'interface coeur-électrode 27.

Lorsque l'interrupteur 28 s'ouvre, l'interrupteur 25 se ferme pendant 12ms, afin de décharger la capacité de sortie 26 et l'interface électrode-coeur 27. Pour éviter une saturation, l'amplificateur de détection de la réponse cardiaque 30 (Fig. 2) est déconnecté pendant la durée de l'impulsion augmentée de 14 ms, par une occultation connue sous le nom de blanking, et symbolisée par l'interrupteur 37.

Sur la Fig. 2, le signal endocavitaire recueilli sur la sonde 31 est amplifié et numérisé par modulation delta dans une chaîne électronique de mesure comportant un amplificateur 30, un comparateur 32, un compteur-décompteur 33, et un convertisseur numérique-analogique 34. Le pas de comptage est appliqué au compteur en 35 et l'état de sortie du compteur 33 est disponible sous forme de code en 36, et lu par un micro-contrôleur qui réalise tous les calculs.

Dans le cadre de l'invention, l'amplitude de stimulation est régulée avec un pas de 0,25 V pour les tensions comprises entre 1,25 et 3 V et avec un pas de 0,5 V entre 3 V et 5 V.

Dans certains cas, on commande une contre-stimulation de secours 64ms après une stimulation qui s'est révélée inefficace. Cette contre-stimulation a une durée, 1ms par exemple, supérieure à la durée de la première stimulation, 0,5ms par exemple, et une amplitude supérieure à l'amplitude de la première stimulation. La recharge de la capacité de stimulation ventriculaire 22 est accélérée dès la délivrance de la première stimulation en suspendant la régulation et la recharge de la capacité de stimulation auriculaire, de façon que l'amplitude de la a tension de charge de la capacité 22, correspondant à la deuxième stimulation, soit au dessus de la valeur précédente.

En fonctionnement standard, après chaque stimulation, sauf en cas de stimulation à haute énergie avec une amplitude de 5V, le signal endocavitaire est analysé pour déterminer l'efficacité de la stimulation, c'est-à-dire pour déterminer si le seuil de capture a été franchi. En cas d'inefficacité, les quatre stimulations des cycles suivantes sont délivrées à haute énergie.

En fonctionnement standard, et en dehors des stimulations à haute énergie qui ne sont pas prises en compte, les stimulations sont comptabilisées au moyen d'un compteur-décompteur à 32 pas de comptage. Ce compteur est initialisé à 31 après chaque recherche de seuil aboutissant à une détermination de la valeur du seuil. A chaque stimulation efficace, le compteur est incrémenté de 1. A chaque stimulation inefficace, le compteur est décrémenté de 4. Lorsque le compteur descend à 0, le stimulateur considère le seuil comme erroné et il déclenche une phase d'étalonnage. Cette situation correspond à un taux d'efficacité insuffisant.

Ainsi, une série de huit stimulations inefficaces successives, chacune suivie par des stimulations à haute énergie, déclenche une recherche de seuil.

Pour déterminer si le seuil de capture a été franchi, un paramètre peut servir de critère de capture si sa valeur permet de différencier les stimulations efficaces des stimulations inefficaces, rapidement et de façon fiable. Selon l'invention, le paramètre choisi est l'amplitude négative maximale du signal cardiaque, obtenue après le blanking et dans les 64ms suivant la stimulation, par rapport à la ligne de base de ce signal.

On a pu observer que la valeur du paramètre augmente brutalement quand on passe d'une stimulation inefficace à une stimulation efficace, pour une énergie de stimulation constante correspondant au seuil d'efficacité.

Pour les stimulations efficaces, le paramètre augmente pratiquement linéairement avec l'énergie de stimulation, c'est-à-dire avec le carré de la tension, la durée des impulsions de stimulation étant fixée.

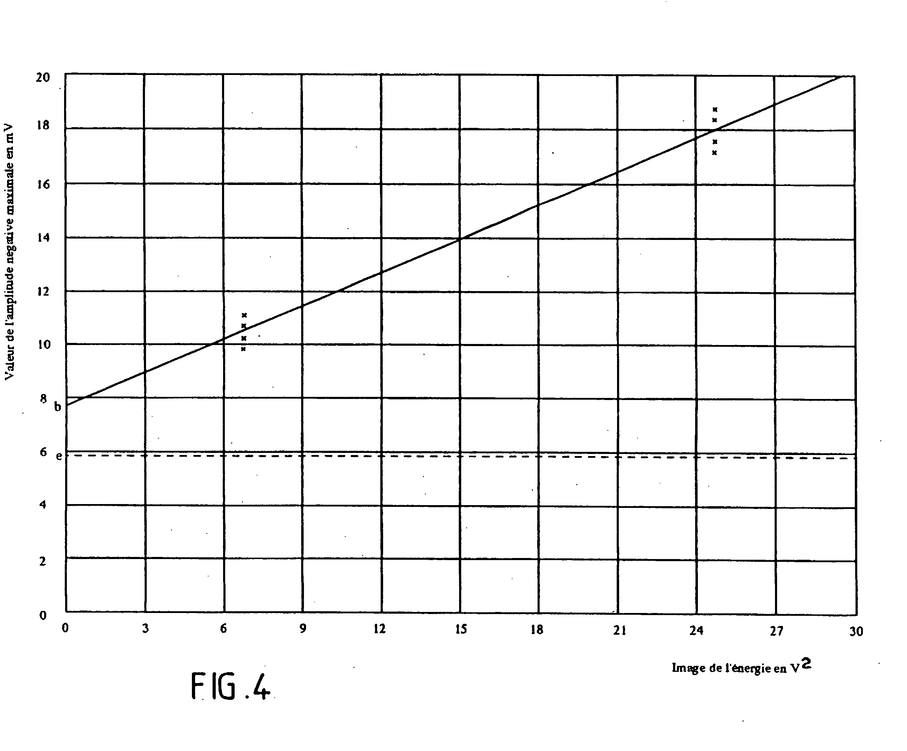

Selon l'invention, le paramètre de capture est mesuré et comparé à une valeur de référence pour statuer sur l'efficacité de la stimulation. La valeur de référence est déterminée au cours de la phase d'étalonnage, en relation avec la Fig. 4, et la mesure du paramètre est effectuée par soustraction entre la valeur négative maximale du signal cardiaque et la ligne de base. Le signal endocavitaire amplifié et numérisé par la chaîne de mesure de la Fig. 2, est échantillonné toutes les ms pendant les 64ms suivant la stimulation. La ligne de base est obtenue en faisant un échantillon entre 2 et 12ms après la stimulation, pour éviter les éventuels régimes transitoires de stimulation, de décharge de la capacité de sortie, et de blanking.

Le calcul de l'amplitude négative maximale commence après la fin du blanking et s'arrête 64ms après la stimulation. Le pas de comptage-décomptage est programmé à 1, mais si la différence entre deux échantillons successifs est supérieure à 1mV avant amplification, le pas de comptage-décomptage est programmé à 2.

Le suivi automatique du seuil de capture est assuré au moyen d'un algorithme qui comporte deux phases spécifiques. Tout d'abord une phase d'étalonnage qui permet de déterminer la valeur de référence du paramètre, et ensuite une phase de recherche de seuil pour mesurer le seuil d'efficacité.

La phase d'étalonnage est lancée à période fixe, par exemple de 24 h en situation chronique et de 6 h en situation aiguë, à condition que les huit cycles précédents aient été stimulés à une période supérieure à la période minimale augmentée de 32ms. Dans le cas contraire, la phase d'étalonnage est différée jusqu'à ce que la condition soit remplie. La phase d'étalonnage est également lancée en cas d'insuffisance du taux d'efficacité.

La phase de recherche de seuil est automatiquement lancée après chaque étalonnage, et à période fixe, par exemple de 6 h en situation chronique et de 1 h 30 en situation aiguë, à condition que les huit cycles précédents aient été stimulés à une période supérieure à la période minimale Tmin augmentée de 32ms. Dans le cas contraire, elle est différée jusqu'à ce que la condition soit remplie.

A chaque déclenchement de la phase d'étalonnage ou de la phase de recherche de seuil, pour éviter le phénomène de fusion, on procède à une réduction de la période cardiaque de 32 ms, sauf dans le cas où le stimulateur fonctionne en mode DDD avec un délai AV inférieur ou égal à 94 ms.

Le phénomène de fusion se caractérise par la simultanéité d'une stimulation et d'une dépolarisation cardiaque spontanée.

Lorsque, au cours de la phase d'étalonnage, la valeur de référence du paramètre correspond à une valeur de seuil supérieure à 2,5V, la phase de recherche de seuil n'est pas déclenchée.

Sur le diagramme d'états de la Fig. 3, les indications ont la signification suivante :
ETALON-5V et ETALON-2,5V : phases d'étalonnage avec des stimulations à 5V et 2,5V respectivement ; calcul de la droite de corrélation paramètre-énergie et de la droite limite de séparation entre stimulations efficaces et inefficaces ;
RECH. SEUIL : séries de quatre stimulations d'amplitudes comprises entre 2,25 V et 1,25 V jusqu'au franchissement du seuil ;
STANDARD : fonctionnement standard ;
   . en cas de seuil valide, l'amplitude et la largeur des impulsions de stimulation sont optimisées en fonction du seuil, et l'efficacité est vérifiée ;
   . en cas de seuil inconnu, ou supérieur ou égal à 2,5 V, les impulsions de stimulation sont à haute énergie, c'est-à-dire qu'elles ont une amplitude de 5 V et une largeur de 0,5 ms à 1ms ;
ATTENTE : fonctionnement standard avec attente des conditions pour passer en phase d'étalonnage ou de recherche de seuil ;
INEFF : après une stimulation inefficace, sont prévues quatre impulsions de stimulation à haute énergie.

Les changements d'états mentionnés sur la Fig. 3 correspondent respectivement à :
1 : demande d'étalonnage ou de recherche de seuil;
2 : stimulation inefficace ;
3 : fin des 4 stimulations à haute énergie ;
4 : demande d'étalonnage et conditions respectées;
5 : demande de recherche de seuil et conditions respectées ;
6 : abandon d'étalonnage ;
7 : fin de la première partie de l'étalonnage ;
8 : fin d'étalonnage et seuil > 2,5 V ;
9 : fin d'étalonnage avec demande de recherche de seuil ou abandon d'étalonnage ;
10 : abandon de recherche de seuil ;
11 : fin de recherche de seuil.

En fonctionnement standard, la stimulation est réalisée avec une amplitude calculée de manière à avoir une marge de sécurité supérieure ou égale à 100% par rapport au seuil d'efficacité. On mesure donc le seuil et on calcule l'amplitude de l'impulsion de stimulation au moins au double de la valeur de seuil. De plus, le seuil est toujours mesuré avec des impulsions dont la largeur est de 0,5ms. Dans le tableau récapitulatif, les valeurs de seuil sont indiquées avec un pas de 0,25V, et les amplitudes correspondantes de l'impulsion de stimulation avec un pas de 0,5V

### TABLEAU RECAPITULATIF

| Seuil en V à 0,5ms | Amplitude en V | Largeur en ms |
|---|---|---|
| | F1 | F2 |
| seuil < 1,25 | 2,5 | 0,5 |
| 1,25 ≤ seuil < 1,50 | 3,0 | 0,5 |
| 1,50 ≤ seuil < 1,75 | 3,5 | 0,5 |
| 1,75 ≤ seuil < 2,00 | 4,0 | 0,5 |
| 2,00 ≤ seuil < 2,25 | 4,5 | 0,5 |
| 2,25 ≤ seuil < 2,50 | 5,0 | 0,5 |
| 2,50 ≤ seuil | 5,0 | (0,5 ;1,0) |

La dernière ligne du tableau récapitulatif correspond aux impulsions de stimulation de haute énergie, dont la durée est comprise entre 0,5 et 1ms, et de préférence égale à 0,5ms.

Lorsque le seuil est inférieur à 2,5V, l'efficacité de chaque stimulation à l'amplitude optimale correspondant au tableau est vérifiée. Si une stimulation est inefficace, le programme prévoit que les stimulations des quatre cycles cardiaques suivants sont des stimulations de haute énergie.

Le taux d'efficacité de stimulation est évalué, et le seuil est considéré comme erroné lorsque le compteur de stimulations, descend à 0. Chaque stimulation efficace applique au compteur un incrément de 1, chaque stimulation inefficace applique au compteur un décrément de 4. Lorsque le compteur est à 0, le taux d'efficacité de stimulation est considéré comme insuffisant.

Le programme commande un étalonnage avec une périodicité déterminée, qui est par exemple de 24 heures en situation chronique et de 6 heures en situation aiguë, et chaque fois que le taux d'efficacité des stimulations est insuffisant.

Dans le cas où le seuil est considéré comme erroné ou inconnu, les stimulations sont programmées à haute énergie, puis un étalonnage et une recherche de seuil sont déclenchés.

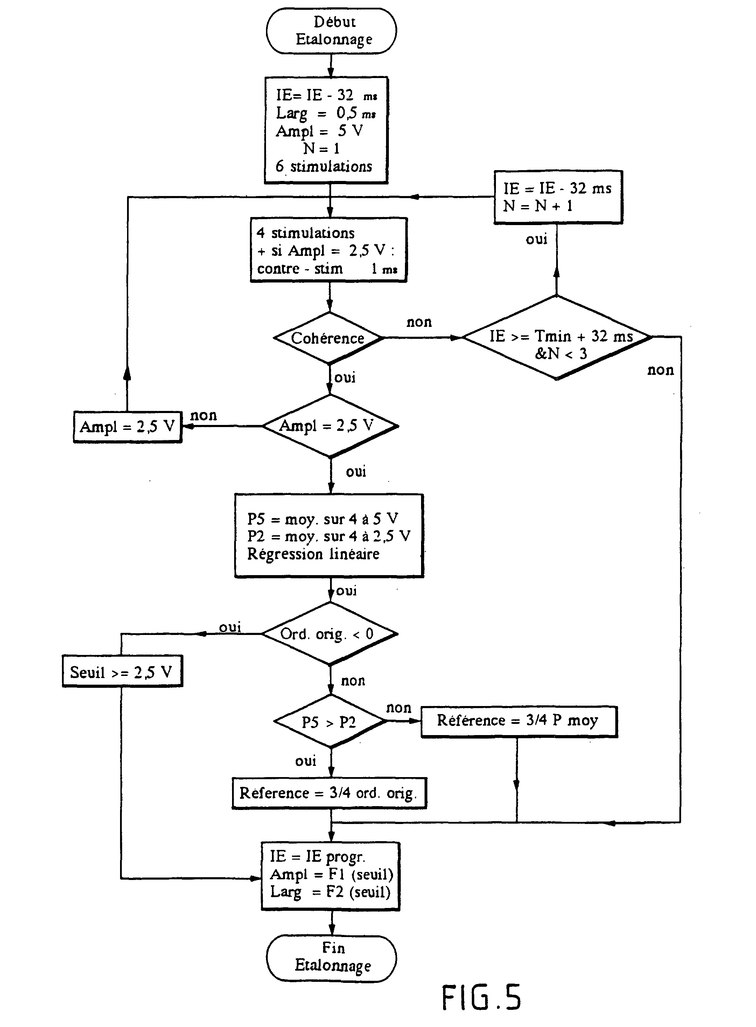

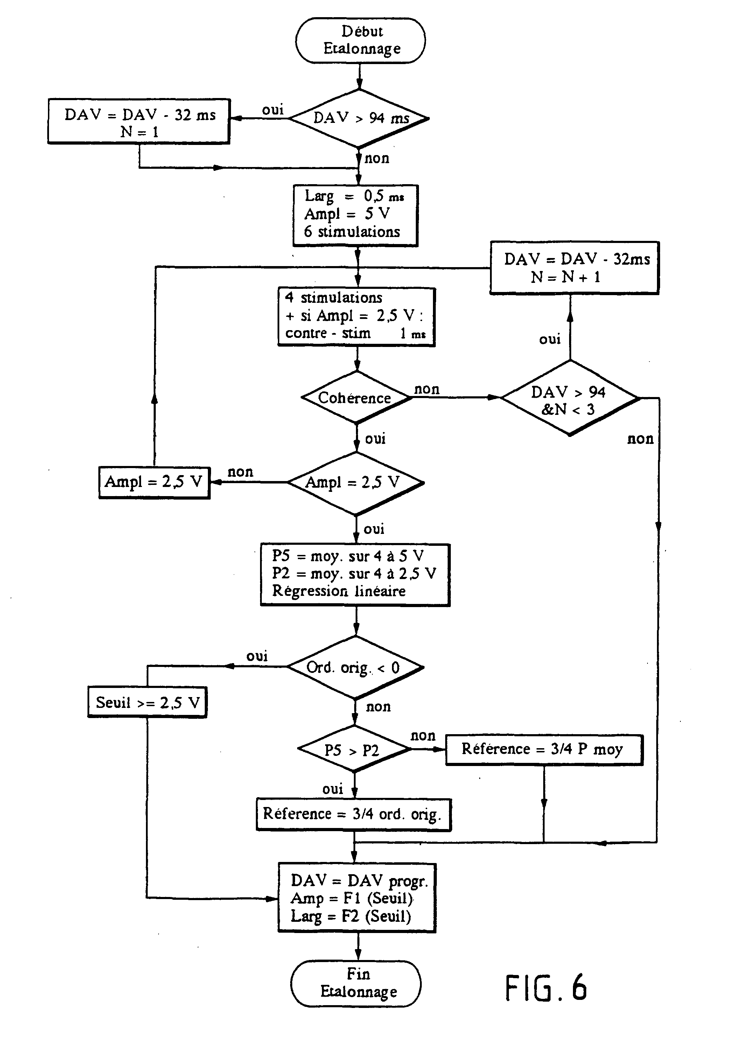

La phase d'étalonnage est décrite en relation avec le diagramme de la Fig. 5 (mode VVI) ou de la Fig. 6 (mode DDD).

L'étalonnage commence après réduction de 32 ms, pour éviter la fusion, soit de l'intervalle d'échappement IE (Fig. 5) en mode VVI, soit du délai AV (Fig. 6) en mode DDD. Si cette réduction n'est pas possible : en mode VVI, on attend que la réduction soit devenue possible, et en mode DDD, on procède à l'étalonnage sans réduction du délai AV.

L'étalonnage consiste à déterminer, pour les stimulations efficaces, la droite représentative du paramètre de capture (Fig. 4) en fonction du carré de l'amplitude de stimulation, qui constitue une image de l'énergie de stimulation.

La détermination de cette droite permet de fixer la limite de la région d'efficacité.

La droite est déterminée par deux points correspondant respectivement aux amplitudes de stimulation 2,5V et 5V, avec une durée de 0,5ms. Après chaque stimulation à 2,5V, une contre-stimulation de sécurité à 1ms est déclenchée pour le cas où le seuil excéderait 2,5V.

Pour chacune des deux amplitudes, une série de quatre stimulations est réalisée et le programme assure la mesure du paramètre et sa moyenne :P2 pour l'amplitude 2,5V; P5 pour l'amplitude 5V.

Dans le cas de l'amplitude 5V, avant la série de quatre stimulations, le programme déclenche six stimulations afin de stabiliser la charge de la capacité de stimulation, ce qui est souhaitable lorsque la pile est en service depuis longtemps.

Lorsque les quatre valeurs du paramètre de capture sont dispersées, c'est-à-dire leur écart relatif important, le programme considère qu'elles ne sont pas cohérentes. Si la période du cycle cardiaque n'est pas supérieure à la période minimale autorisée Tmin augmentée de 32 ms (en mode VVI), ou si le délai AV n'est pas supérieur à 94 ms (en mode DDD), le programme sort de la phase d'étalonnage. Si la période du cycle cardiaque est supérieure à la période minimale autorisée Tmin augmentée de 32 ms et si le stimulateur fonctionne en mode VVI, l'intervalle d'échappement IE ventriculaire est diminué de 32ms et une nouvelle série de quatre stimulations est déclenchée. Si le stimulateur fonctionne en mode DDD et si le délai auriculo-ventriculaire est supérieur à 94 ms, le délai AV est réduit de 32 ms et une nouvelle série de quatre stimulations est déclenchée. Cette opération est réalisée pour des séries de quatre impulsions dont le nombre N est, au maximum, égal à 3. Si les valeurs du paramètre sont toujours non cohérentes, le programme sort de la phase d'étalonnage.

Les valeurs moyennes du paramètre de capture obtenues pour les amplitudes 5V et 2,5V permettent le calcul de la pente a et de l'ordonnée à l'origine b de la droite (y = ax + b) représentative du paramètre en fonction du carré de l'amplitude (Fig. 4).

Le programme fixe alors la valeur de référence e du paramètre de capture par exemple, aux trois quarts de la valeur b, de façon à éliminer les stimulations inefficaces considérées comme efficaces. L'erreur contraire consistant à considérer comme inefficace une stimulation efficace n'est pas préjudiciable.

Dans le cas où la dispersion des mesures conduit à une pente négative pour la droite, le programme considère la pente comme nulle et prend pour valeur b la moyenne des valeurs du paramètre pour les deux amplitudes de stimulation (Pmoy).

Dans le cas où la valeur b calculée est très faible ou négative, le programme considère que le seuil est supérieur à 2,5 V : la valeur de référence du paramètre n'est pas calculée et des stimulations à haute énergie sont prévues.

Pendant le déroulement de la phase d'étalonnage, la détection d'un complexe spontané provoque le report de la stimulation au cycle cardiaque suivant. La 32ème détection provoque l'arrêt de la phase d'étalonnage et sa suspension jusqu'à l'occurrence de huit cycles stimulés consécutifs.

Lorsque la phase d'étalonnage définit pour le seuil une valeur supérieure à 2,5 V, la phase de recherche de seuil n'est pas déclenchée.

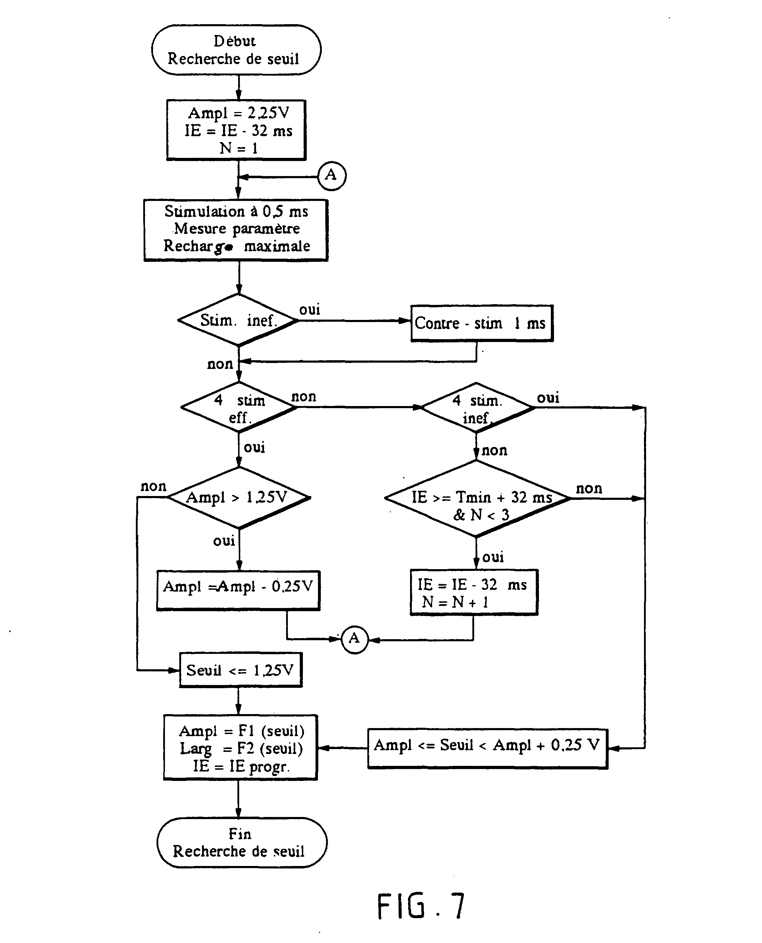

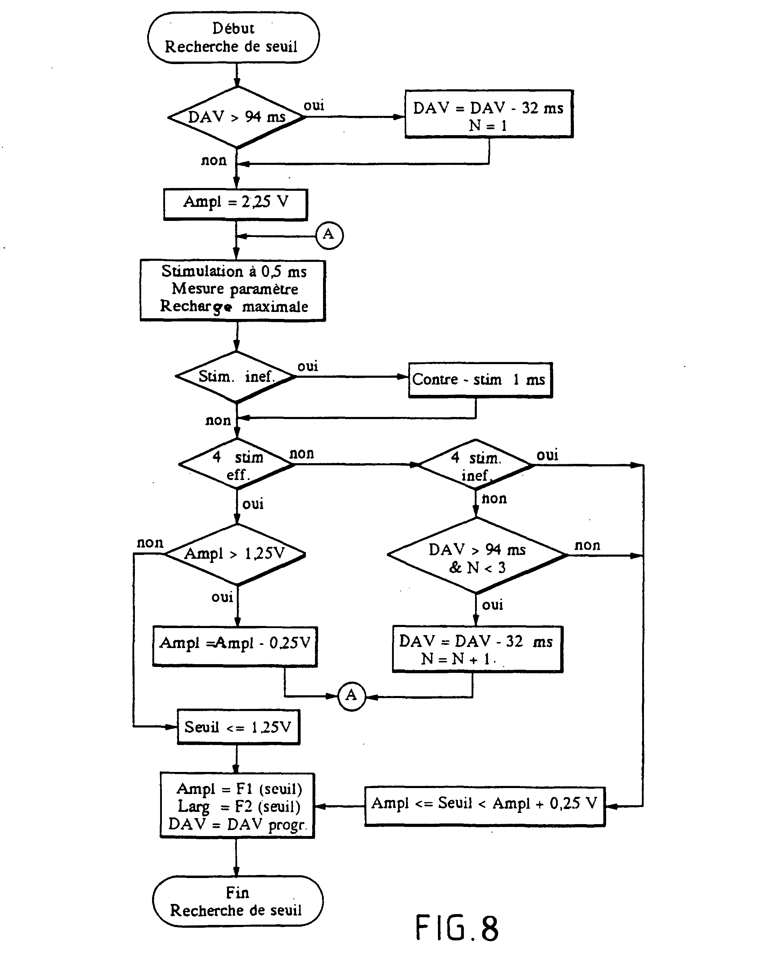

Après la phase d'étalonnage, le programme lance la phase de recherche de seuil correspondant au diagramme de la Figure 7 (mode VVI) ou de la Fig. 8 (mode DDD).

Le seuil d'efficacité est recherché avec des séries de quatre impulsions de largeur 0,5 ms et dont l'amplitude varie à chaque série, de 2,25 V à 1,25 V.

Entre chaque série de quatre impulsions, l'amplitude est réduite de 0,25 V.

L'efficacité est déterminée pour chaque stimulation. Lorsqu'une stimulation est inefficace, une contre-stimulation de 1 ms est déclenchée à la fin du délai de 64 ms.

Après chaque série de quatre stimulations à même énergie, trois cas se présentent :
1/ **Les quatre stimulations sont efficaces.**
   Si l'amplitude est alors de 1,25 V, la recherche de seuil est arrêtée et le seuil de 1,25 V est retenu. Si l'amplitude est supérieure à 1,25 V, une nouvelle série de quatre impulsions est déclenchée, avec une amplitude réduite de 0,25 V.
2/ **Les quatre stimulations sont inefficaces.**
   La recherche de seuil est arrêtée et le seuil retenu est égal à l'amplitude de la série précédente de quatre stimulations.
3/ **Les quatre stimulations ne donnent pas toutes le même résultat.**

Si la période du cycle cardiaque est supérieure à la période minimale augmentée de 32 ms et si le stimulateur est en mode VVI, l'intervalle d'échappement ventriculaire IE est diminué de 32 ms pour réduire le risque de fusion, et une série de quatre impulsions est déclenchée avec la même amplitude. Le nombre de réductions de IE est, dans ce cas, limité à trois.

Si le stimulateur est en mode DDD est si le délai AV est supérieur à 94 ms, ce délai AV est réduit de 32 ms et une série de quatre impulsions est déclenchée avec la même amplitude. Le nombre de réductions du délai AV est, dans ce cas, limité à trois.

Sinon, la valeur retenue pour le seuil est celle de l'amplitude précédente.

Au cours de la phase de recherche de seuil, la détection d'un complexe spontané provoque le report de la stimulation au cycle cardiaque suivant, et la 32ème détection provoque l'arrêt de la recherche de seuil jusqu'à l'occurrence de huit cycles stimulés consécutifs.

Après détermination du seuil de capture, le programme prévoit la définition de l'amplitude F1 de l'impulsion de stimulation selon le tableau récapitulatif précédent.

Le procédé selon l'invention est de préféfence, exécuté par un dispositif à microprocesseur possédant des instructions de logiciel programmées pour exécuter les étapes de procédé décrites plus haut.

L'élaboration du logiciel adéquat pour contrôler des stimulateurs cardiaques contrôlés par microprocesseur peut être effectuée par un homme du métier de compétence ordinaire. Bien entendu, il est également possible de mettre en oeuvre la présente invention au moyen de circuits analogiques ou numériques de type connu.

Grâce à l'invention, le réglage de l'énergie de stimulation ventriculaire selon l'invention permet d'adapter automatiquement et périodiquement l'énergie de stimulation aux besoins du patient.

Les avantages de la présente invention sont en particulier :
- une plus grande sécurité pour le patient car le stimulateur s'adapte à l'évolution du seuil de capture ;
- une diminution des contraintes pour le médecin qui n'a plus à effectuer de test préalable ni de réglage des paramètres de stimulation ;
- une réduction de la consommation d'énergie, c'est-à-dire une réduction de la taille de la pile et/ou une augmentation de sa durée de vie.

L'invention relative au réglage de l'énergie de stimulation s'applique à un stimulateur simple chambre, ou double chambre comportant une stimulation ventriculaire avec amplitude régulée et une détection ventriculaire avec un amplificateur à récupération rapide pour détecter la réponse cardiaque.

Les valeurs numériques sont données à titre d'exemple dans la description qui précède : elles sont susceptibles d'adaptation et n'ont aucun caractère limitatif.

Les avantages du réglage de l'énergie de stimulation selon l'invention sont notamment les suivants:
- l'utilisation d'une sonde classique, unipolaire ou bipolaire, car l'invention fonctionne quel que soit le type de sonde ;
- une polarité pour la détection de la capture qui est la même que la polarité de détection (on entend par polarité le caractère unipolaire ou bipolaire de la sonde);
- l'absence de commutation, ou de modification des caractéristiques de l'amplificateur ;
- l'absence d'application de charge opposée à la charge de stimulation, et la simple décharge de la capacité de sortie pendant un intervalle de temps de 12 ms ;
- la détection de la capture dans un délai de 14 à 64 ms après la stimulation ;
- l'application d'une contre-stimulation 64 ms après la stimulation, en cas de stimulation inefficace, pendant la phase d'étalonnage et pendant la phase de recherche de seuil ;
- l'application de l'invention aussi bien au cas de la stimulation ventriculaire (mode VVI) qu'au cas de la stimulation auriculaire (mode AAI).

Par ailleurs, dans l'exemple de la Fig. 4, on a retenu pour simplifier le cas d'une caractéristique droite. Le procédé est aussi applicable au cas d'une caractéristique courbe en prenant plusieurs points pour la phase d'étalonnage, et retenir pour valeur de b l'ordonnée à l'origine de la courbe extrapolée.

## Revendications

1. Procédé de contrôle au moyen d'un logiciel, par la mesure d'un paramètre de capture, de l'énergie de stimulation dans un stimulateur cardiaque, comprenant les étapes suivantes :
- un paramètre de capture est défini constitué par l'amplitude négative maximale du signal cardiaque obtenu après la stimulation, dans un délai déterminé, en fonction de l'énergie de stimulation;
- les caractéristiques représentatives du paramètre de capture en fonction de l'énergie de stimulation sont déterminées au cours d'une phase d'étalonnage ;
- une valeur de référence pour déterminer l'efficacité des stimulations est définie à partir de ces caractéristiques ;
- la valeur du seuil de capture est déterminée à partir de la valeur de référence au cours d'une phase de recherche de seuil ;
- et l'énergie de stimulation est déterminée en fonction de la valeur du seuil de capture.

2. Procédé selon la revendication 1, **caractérisé en ce que** le délai déterminé commence après le blanking et se termine environ 64 ms après la stimulation.

3. Procédé selon la revendication 1, **caractérisé en ce que** dans le cas où le paramètre de capture varie linéairement en fonction de l'énergie de stimulation, les caractéristiques de la droite représentative du paramètre de capture sont la pente (a) et l'ordonnée à l'origine (b).

4. Procédé selon la revendication 3, **caractérisé en ce que** la valeur de référence pour déterminer l'efficacité des stimulations est une fraction de l'ordonnée à l'origine (b) égale de préférence à 3/4.

5. Procédé selon la revendication 1, **caractérisé en ce qu'**au commencement de la phase d'étalonnage ou de la phase de recherche de seuil, on diminue, si cela a est possible, la période du cycle cardiaque de façon à éviter le phénomène de fusion.

6. Procédé selon la revendication 1, **caractérisé en ce que** pour déterminer la valeur du seuil de capture, des séries successives d'impulsions de stimulation sont produites en faisant décroître l'amplitude des impulsions entre les séries.

7. Procédé selon la revendication 6, **caractérisé en ce que** chaque série d'impulsions est constituée de préférence de quatre impulsions de même amplitude.

8. Procédé selon la revendication 6, **caractérisé en ce que**, après chaque série d'impulsions toutes efficaces, on fait décroître l'amplitude d'une quantité déterminée, de préférence 0,25 V.

9. Procédé selon la revendication 8, **caractérisé en ce que**, lorsque l'amplitude des impulsions est de l'ordre de 1,25V, on garde cette valeur comme valeur de seuil.

10. Procédé selon la revendication 6, **caractérisé en ce que**, après une série d'impulsions toutes inefficaces, on définit comme valeur de seuil l'amplitude des impulsions de la série précédente.

11. Procédé selon la revendication 6, **caractérisé en ce que**, si le stimulateur fonctionne en mode VVI, après une série d'impulsions de stimulation ne donnant pas toutes le même résultat, on vérifie si la période du cycle cardiaque est supérieure d'au moins une durée d'environ 32ms à la période minimale autorisée : dans ce cas, on réduit de ladite durée, l'intervalle d'échappement ventriculaire et on procède à une nouvelle série d'impulsions avec la même amplitude, et dans le cas contraire on définit comme valeur de seuil l'amplitude des impulsions de la série précédente.

12. Procédé selon la revendication 6, **caractérisé en ce que**, si le stimulateur fonctionne en mode DDD, après une série d'impulsions ne donnant pas toutes le même résultat, on vérifie si le délai AV est supérieur d'au moins une durée d'environ 32ms à une valeur de préférence égale à 62ms : dans ce cas, on réduit le délai AV de ladite durée, et on procède à une nouvelle série d'impulsions avec la même amplitude, et dans le cas contraire on définit comme valeur de seuil l'amplitude des impulsions de la série précédente.

13. Procédé selon la revendication 1, **caractérisé en ce que** l'énergie de stimulation est déterminée en prenant pour amplitude des impulsions de stimulation le double de la valeur du seuil de capture.

14. Procédé selon la revendication 1, **caractérisé en ce que**, pendant la phase d'étalonnage, après chaque stimulation à 2,5 V, on déclenche une contre stimulation.

15. Procédé selon la revendication 1, **caractérisé en ce que**, pendant la phase de recherche de seuil, en cas de stimulation inefficace, on déclenche une contre-stimulation.

16. Procédé selon la revendication 1, **caractérisé en ce que**, en dehors des phases d'étalonnage et de recherche de seuil, sauf en cas de stimulation à haute énergie, on vérifie l'efficacité de chaque stimulation, et en cas de stimulation inefficace, les stimulations des cycles suivants, de préférence au nombre de quatre, sont délivrées à haute énergie.

17. Procédé selon la revendication 1, **caractérisé en ce qu'**on évalue le taux d'efficacité des stimulations au moyen d'un compteur-décompteur auquel on applique un incrément de 1, de préférence, pour chaque stimulation efficace, et un décrément de 4, de préférence, pour chaque stimulation inefficace, et lorsque le compteur descend à 0, on définit le taux d'efficacité des stimulations comme insuffisant, et on déclenche une phase d'étalonnage ;

18. Procédé selon la revendication 1, **caractérisé en ce que**, pendant la phase d'étalonnage on vérifie de préférence au maximum trois fois, la cohérence des séries de stimulations.

19. Procédé selon l'une des revendications 11 ou 12, **caractérisé en ce que**, pendant la phase de recherche de seuil, on procède de préférence au maximum trois fois à la réduction de la période cardiaque pour la détermination de l'efficacité des stimulations.

20. Procédé selon la revendication 1, **caractérisé en ce qu'**on distingue une situation chronique, et une situation aiguë correspondant à une période d'environ un mois à compter de l'implantation du stimulateur.

21. Procédé selon la revendication 20, **caractérisé en ce que** la périodicité des phases d'étalonnage est de 24 heures en situation chronique et de 6 heures en situation aiguë.

22. Procédé selon la revendication 20, **caractérisé en ce que** la périodicité des phases de recherche de seuil est de 6 heures en situation chronique et de 1 heure 30 en situation aiguë.

23. Procédé selon la revendication 1, **caractérisé en ce que**, lorsque la phase d'étalonnage définit pour le seuil une valeur supérieure à 2,5V, la phase de recherche de seuil n'est pas déclenchée.

24. Procédé de contrôle selon la revendication 1, de l'énergie de stimulation d'une impulsion de stimulation ayant une amplitude et une largeur pour stimuler, fournies par un stimulateur cardiaque, **caractérisé par** les étapes suivantes:
- surveiller les signaux endocavitaires en réponse à ladite impulsion de stimulation fournie par le stimulateur et fournir ladite valeur de réponse représentative de la réponse cardiaque;
- définir le seuil de capture correspondant à une énergie minimale d'impulsion de stimulation produisant une valeur désirée de réponse cardiaque ;
- contrôler l'énergie d'impulsion de stimulation correspondant à la valeur définie du seuil de capture ;
- définir qu'une impulsion de stimulation fournie est efficace si la valeur fournie de la réponse cardiaque est supérieure au seuil de capture défini, et inefficace si la valeur fournie de la réponse cardiaque est inférieure au seuil de capture défini, et
- produire un nombre choisi d'impulsions de stimulations à haute énergie en réponse à une impulsion de stimulation définie comme inefficace.

25. Procédé selon la revendication 24, **caractérisé en ce qu'**en outre : on tient un compte des impulsions de stimulation efficaces et inefficaces, on incrémente le compte chaque fois que l'impulsion de stimulation fournie est efficace ; on décrémente le compte à chaque fois que l'impulsion de stimulation fournie est inefficace ; et on définit un nouveau seuil de capture en réponse à une diminution du compte jusqu'à une valeur de compte prédéterminée.

26. Procédé selon la revendication 25, **caractérisé en ce qu'**on applique un incrément de un au compte pour chaque impulsion de stimulation définie comme efficace jusqu'à une valeur maximale du compte et on applique un décrément de quatre pour chaque impulsion de stimulation définie comme inefficace, et **en ce que** ladite valeur de compte prédéterminée est zéro.

27. Procédé selon la revendication 24, **caractérisé en ce que** la définition du seuil de capture comporte en outre les étapes suivantes :
- produire une pluralité d'impulsions de stimulation d'étalonnage ayant une amplitude et une largeur déterminées;
- déterminer si les impulsions de stimulation d'étalonnage sont efficaces ou non ;
- ajuster l'amplitude des impulsions de stimulation d'étalonnage pour identifier l'amplitude minimale d'impulsion pour la largeur donnée correspondant à une impulsion de stimulation efficace, et
- définir le seuil de capture à partir de l'amplitude minimale identifiée des impulsions de stimulation d'étalonnage.

28. Procédé selon la revendication 24, **caractérisé en ce que** la définition du seuil de capture comporte en outre les étapes suivantes :
i. produire une série d'un premier nombre d'impulsions de stimulation d'étalonnage ayant une largeur et une amplitude déterminées ;
ii. déterminer si chaque impulsion du premier nombre d'impulsions de la série produite est efficace, et
iii. en réponse au fait que chaque impulsion du premier nombre d'impulsions de la série est efficace,
a) réduire l'amplitude de l'impulsion de stimulation d'étalonnage d'une quantité prédéterminée et produire une autre série du premier nombre d'impulsions de stimulation d'étalonnage à l'amplitude d'impulsion réduite;
b) déterminer si chaque impulsion du premier nombre d'impulsions de la série produite est efficace ;
c) en réponse au fait que chaque impulsion du premier nombre d'impulsions est inefficace, définir le seuil de capture comme étant l'amplitude des impulsions de stimulation d'étalonnage de la série précédente, et,
d) en réponse au fait qu'une impulsion du premier nombre d'impulsions est efficace, recommencer l'étape iii.

29. Procédé selon la revendication 28, **caractérisé en ce que** le premier nombre d'impulsions est quatre, et **en ce que** chaque impulsion a la même largeur et la même amplitude.

30. Procédé selon la revendication 28, **caractérisé en ce que** la largeur d'impulsion est choisie entre 0,5 ms et 1,0 ms et la quantité déterminée est choisie entre 0,25 et 0,5 Volts.

31. Procédé selon la revendication 24, **caractérisé en ce que** la définition du seuil de capture comporte : une étape consistant à maintenir le seuil de capture défini à une valeur égale ou supérieure à un seuil de capture minimal correspondant à une amplitude et une largeur d'impulsion minimales.

32. Procédé selon la revendication 28, dans lequel le stimulateur fonctionne en mode VVI avec un intervalle d'échappement ventriculaire et un cycle cardiaque, **caractérisé en ce que** l'étape ii, comporte en outre : déterminer si la série d'impulsions de stimulation comporte au moins une impulsion de stimulation d'étalonnage efficace et une impulsion de stimulation d'étalonnage inefficace, et en réponse, déterminer si la période du cycle cardiaque analysé est supérieure d'au moins une première durée à une période minimale déterminée, et, dans ce cas, réduire de la première durée, l'intervalle d'échappement ventriculaire et recommencer les étapes i-iii avec des impulsions de stimulation d'étalonnage avec la même amplitude que la série précédente et, dans le cas contraire, définir la valeur de seuil de capture comme étant l'amplitude des impulsions de la série précédente.

33. Procédé selon la revendication 28, dans lequel le stimulateur fonctionne en mode DDD avec un délai AV, **caractérisé en ce que** l'étape ii comporte en outre: déterminer si la première série d'impulsions de stimulation d'étalonnage comporte au moins une impulsion efficace et au moins une impulsion inefficace, et, en réponse, déterminer si le délai AV est supérieur d'au moins une première quantité à une valeur prédéterminée et, dans ce cas, réduire le délai AV de la première quantité et recommencer les étapes i-iii avec des impulsions de stimulation d'étalonnage à la même amplitude d'impulsion qu'au cycle précédent, et, dans le cas contraire, définir la valeur de seuil de capture comme étant l'amplitude des impulsions de la série précédente.

34. Procédé selon la revendication 24, **caractérisé en ce que** l'étape de définition du seuil de capture comprend en outre : la détermination d'un nouveau seuil de capture selon une périodicité d'environ 24 heures en situation chronique et d'environ 6 heures en situation aiguë.

35. Procédé selon la revendication 24, **caractérisé en ce que** l'étape de définition du seuil de capture comporte en outre :
· définir un paramètre de capture qui varie en fonction de l'énergie de stimulation :
· prévoir une phase d'étalonnage pour déterminer des caractéristiques représentatives du paramètre de capture en fonction de la réponse cardiaque analysée à une ou plusieurs impulsions de stimulation d'étalonnage ;
· définir une valeur de référence pour déterminer l'efficacité d'impulsions de stimulation d'étalonnage en réponse aux caractéristiques représentatives déterminées du paramètre de capture ; et
· prévoir une phase de recherche de seuil pour déterminer la valeur du seuil de capture par rapport à la valeur de référence définie.

36. Dispositif de contrôle de l'énergie de stimulation dans un stimulateur cardiaque, comportant :
- un générateur d'impulsions pour produire des impulsions ayant une énergie de stimulation déterminée,
- un moniteur pour détecter la réponse cardiaque à l'énergie de stimulation d'une impulsion fournie,
- des moyens de commande de l'énergie de stimulation fournie par rapport à une valeur déterminée de seuil de capture,
**caractérisé en ce qu'**il comporte des moyens pour définir le seuil de capture selon une procédure d'étalonnage dans laquelle des caractéristiques représentatives d'un paramètre de capture constitué par l'amplitude négative maximale du signal cardiaque obtenu après la stimulation dans un délai déterminé, sont déterminées en fonction de l'énergie de stimulation analysée en réponse à une ou plusieurs impulsions, le paramètre de capture variant en fonction de l'énergie de stimulation, et dans laquelle une valeur de référence est définie pour déterminer l'efficacité d'une impulsion en réponse aux caractéristiques représentatives déterminées du paramètre de capture, et selon une procédure de recherche de seuil dans laquelle la valeur du seuil de capture est déterminée sur la base de la valeur de référence définie.

37. Dispositif selon la revendication 36 de contrôle de l'énergie de stimulation d'impulsions de stimulation fournies par un stimulateur cardiaque, **caractérisé en ce que** :
- le générateur produit des impulsions de stimulation ayant une amplitude et une largeur pouvant être définies et correspondant à une énergie de stimulation ;
- la moniteur détecte la réponse endocavitaire aux impulsions de stimulation fournies par le stimulateur et fournit une valeur de réponse représentative de la réponse cardiaque ;
et **en ce qu'**il comporte :
- des moyens pour définir le seuil de capture pour correspondre à l'énergie minimale d'impulsion de stimulation produisant une valeur déterminée de réponse cardiaque, et
- des moyens pour déterminer que l'impulsion de stimulation fournie est efficace lorsque la valeur de la réponse cardiaque est supérieure au seuil de capture défini et inefficace lorsque la valeur fournie de la réponse cardiaque est inférieure au seuil de capture défini, de sorte que les moyens de commande fassent produire par le générateur un nombre déterminé d'impulsions de stimulation ayant une amplitude et une largeur déterminées correspondant à une impulsion de haute énergie en réponse à une impulsion de stimulation définie comme inefficace.

38. Dispositif selon la revendication 37, **caractérisé en ce qu'**il comprend en outre : un compteur pour tenir un compte des impulsions de stimulation efficaces et inefficaces, le compteur étant incrémenté lorsque l'impulsion de stimulation fournie est efficace et décrémenté lorsque l'impulsion de stimulation fournie est inefficace, de sorte que les moyens de définition définissent un nouveau seuil de capture lorsque le compteur descend à une valeur de compte prédéterminée.

39. Dispositif selon la revendication 38, **caractérisé en ce que** le compteur est incrémenté de un pour chaque impulsion de stimulation déterminée comme efficace jusqu'à une valeur maximale de compte, et décrémenté de quatre pour chaque impulsion de stimulation déterminée comme inefficace, et **en ce que** la valeur prédéterminée de compte est zéro.

40. Dispositif selon la revendication 37, **caractérisé en ce que** les moyens de définition comportent en outre : des moyens agissant sur le générateur pour produire une pluralité d'impulsions de stimulation d'étalonnage ayant une amplitude et une largeur déterminées; un comparateur pour déterminer si les impulsions de stimulation d'étalonnage sont efficaces ou non : de sorte que les moyens agissant sur le générateur ajustent l'amplitude des impulsions de stimulation d'étalonnage pour identifier l'amplitude minimale d'impulsion correspondant à une impulsion de stimulation efficace pour une largeur d'impulsion donnée ; et que les moyens de définition définissent le seuil de capture à partir de l'amplitude minimale identifiée des impulsions de stimulation d'étalonnage.

41. Dispositif selon la revendication 37, **caractérisé en ce que** les moyens de définition comportent en outre :
- des moyens agissant sur le générateur pour produire une série d'un premier nombre d'impulsions de stimulation d'étalonnage ayant une largeur et une amplitude déterminées ;
- des moyens pour déterminer si chacune du premier nombre d'impulsions de la série générée est efficace, de sorte que, en réponse au fait que chaque impulsion du premier nombre est efficace, les moyens agissant font produire au générateur une série d'impulsions de stimulation d'étalonnage ayant une amplitude réduite d'une quantité prédéterminée par rapport à l'amplitude d'impulsions de la série précédente, et les moyens de détermination déterminent si chacune du premier nombre d'impulsions de la série générée est efficace, et de sorte que, en réponse au fait que chacune du premier nombre d'impulsions est inefficace, les moyens de définition définissent le seuil de capture comme étant l'amplitude des impulsions de stimulation d'étalonnage de la série précédente d'impulsions ; et de sorte que les moyens agissant, en réponse au fait que l'une du premier nombre d'impulsions est efficace, font que le générateur et les moyens de détermination continuent à produire des séries successives d'impulsions à amplitude de plus en plus réduite jusqu'à ce que les moyens de définition définissent un seuil de capture.

42. Dispositif selon la revendication 41, **caractérisé en ce que** le premier nombre d'impulsions est quatre et **en ce que** chaque impulsion de la même série a la même largeur et la même amplitude.

43. Dispositif selon la revendication 42, **caractérisé en ce que** la largeur d'impulsion est comprise entre 0,5ms et 1.0ms et la quantité prédéterminée est comprise entre 0,25 et 0,5V.

44. Dispositif selon la revendication 37, **caractérisé en ce que** les moyens de définition comportent en outre un comparateur pour comparer la valeur fournie de la réponse cardiaque à un seuil de capture minimal déterminé, et pour fournir le seuil de capture à la valeur minimale déterminée si la valeur fournie de la réponse cardiaque n'est pas plus grande que la valeur minimale déterminée.

45. Dispositif selon la revendication 41, dans lequel le stimulateur fonctionne en mode VVI avec un intervalle d'échappement ventriculaire, **caractérisé en ce qu'**il comporte en outre :
- des premiers moyens pour déterminer si les impulsions de stimulation d'étalonnage de la série produisent au moins une impulsion de stimulation d'étalonnage efficace et une impulsion de stimulation d'étalonnage inefficace ;
- des seconds moyens pour déterminer si la période d'un cycle cardiaque analysé est supérieure d'au moins une première durée à une période minimale déterminée ; et
- des moyens pour réduire l'intervalle d'échappement ventriculaire d'une première durée en réponse à des impulsions de stimulation d'étalonnage produisant au moins une impulsion de stimulation efficace et une impulsion inefficace lorsque le cycle cardiaque mesuré est supérieur d'au moins une première durée à la période minimale déterminée, de sorte que, en réponse à une réduction de l'intervalle d'échappement ventriculaire, les moyens agissant sur le générateur font produire au générateur une série d'impulsions de stimulation d'étalonnage d'amplitude d'impulsion identique à celle de la série précédente, et, en réponse à l'absence de réduction de l'intervalle d'échappement ventriculaire, les moyens de définition définissent la valeur de seuil comme égale à l'amplitude des impulsions de stimulation d'étalonnage de la série précédente.

46. Dispositif selon la revendication 41, dans lequel le stimulateur fonctionne en mode DDD avec un délai AV, **caractérisé en ce qu'**il comporte en outre :
- des premiers moyens pour déterminer si la série produit au moins une impulsion efficace et une impulsion inefficace de stimulation d'étalonnage ;
- des moyens pour déterminer si le délai AV est supérieur d'une première quantité à une valeur déterminée ; et
- des moyens pour réduire le délai AV de la première quantité dans le cas où le délai AV est supérieur de la première quantité à la valeur déterminée, de sorte que, en réponse à une réduction du délai AV, les moyens agissant sur le générateur font produire au générateur une série d'impulsions de stimulation d'étalonnage d'amplitude identique à celle de la série précédente, et, en réponse à l'absence de réduction du délai AV, les moyens de définition définissent la valeur de seuil comme égale à l'amplitude des impulsions de stimulation d'étalonnage de la série précédente.

47. Dispositif selon la revendication 37, **caractérisé en ce que** les moyens de définition définissent périodiquement un nouveau seuil de capture selon une périodicité d'environ 24 heures en situation chronique et d'environ 6 heures en situation aiguë.

48. Dispositif selon la revendication 37, **caractérisé en ce que** les moyens de définition comportent en outre des moyens pour déterminer le seuil de capture selon une procédure d'étalonnage, de sorte que des caractéristiques représentatives d'un paramètre de capture sont déterminées en fonction de la réponse cardiaque analysée à une ou plusieurs impulsions de stimulation d'étalonnage, le paramètre de capture variant en fonction de l'énergie de stimulation, et de sorte qu'une valeur de référence est définie pour déterminer l'efficacité des impulsions de stimulation en réponse aux caractéristiques représentatives déterminées du paramètre de capture ; et selon une procédure de recherche de seuil, de sorte que la valeur du seuil de capture est déterminée sur la base de la valeur de référence déterminée.

## Patentansprüche

1. Verfahren zur Regelung einer Stimulationsenergie in einem Herzschrittmacher mittels einer Software durch eine Erfassung eines Erfassungsparameters, wobei das Verfahren Schritte umfasst zum
- Definieren eines Erfassungsparameters, der entsprechend der Stimulationsenergie durch die maximale negative Amplitude eines Herzsignals gebildet ist, das nach der Stimulation in einer bestimmten Zeitspanne erhalten wird,
- Bestimmen von repräsentativen Merkmalen des Erfassungsparameters entsprechend der Stimulationsenergie im verlauf einer Abgleichphase;
- Definieren eines Referenzwerts zur Bestimmung der Wirksamkeit der Stimulationen ausgehend von den Merkmalen;
- Bestimmen eines Erfassungsschwellenwerts ausgehend von dem Referenzwert im Verlauf einer Schwellenwertsuchphase;
- und Bestimmen der Stimulationsenergie entsprechend dem Erfassungsschwellenwert.

2. Verfahren nach Anspruch 1, **dadurch gekennzeichnet, dass** die bestimmte Zeitspanne nach einem Austasten beginnt und ungefähr 64 ms nach der Stimulation endet.

3. Verfahren nach Anspruch 1, **dadurch gekennzeichnet, dass** in dem Fall, dass der Erfassungsparameter entsprechend der Stimulationsenergie linear variiert, die Merkmale der für den Erfassungsparameter repräsentativen Geraden die Steigung (a) und die Ursprungs-Ordinate (b) sind.

4. Verfahren nach Anspruch 3, **dadurch gekennzeichnet, dass** der Referenzwert zur Bestimmung der Wirksamkeit der Stimulationen ein Bruchteil der Ursprungs-Ordinate (b), vorzugsweise gleich 3/4 ist.

5. Verfahren nach Anspruch 1, **dadurch gekennzeichnet, dass** zu Beginn der Abgleichphase oder der Schwellenwertsuchphase der Zeitraum des Herzzyklus möglichst so verringert wird, dass das Phänomen der Fusion vermieden wird.

6. Verfahren nach Anspruch 1, **dadurch gekennzeichnet, dass** zur Bestimmung des Erfassungsschwellenwerts aufeinanderfolgende Reihen von Stimulationsimpulsen erzeugt werden, wobei die Amplitude der Impulse zwischen den Reihen verringert wird.

7. Verfahren nach Anspruch 6, **dadurch gekennzeichnet, dass** jede Impulsreihe vorzugsweise aus 4 Impulsen der gleichen Amplitude gebildet wird.

8. Verfahren nach Anspruch 6, **dadurch gekennzeichnet, dass** nach jeder Reihe von vollständig wirksamen Impulsen die Amplitude um einen bestimmten Betrag, vorzugsweise 0,25 V verringert wird.

9. Verfahren nach Anspruch 8, **dadurch gekennzeichnet, dass** der Wert als Schwellenwert beibehalten wird, wenn die Amplitude der Impulse in der Größenordnung von 1,25 V liegt.

10. Verfahren nach Anspruch 6, **dadurch gekennzeichnet, dass** nach einer Reihe von vollständig unwirksamen Impulsen die Amplitude der Impulse der vorherigen Reihe als Schwellenwert definiert wird.

11. Verfahren nach Anspruch 6, **dadurch gekennzeichnet, dass**, wenn der Schrittmacher in einer VVI-Betriebsart arbeitet, nach einer Reihe von Stimulationsimpulsen, die nicht alle zum gleichen Ergebnis führen, überprüft wird, ob die Zeitdauer des Herzzyklus um zumindest eine Dauer von ungefähr 32 ms größer ist als die minimal zulässige Zeitdauer: in diesem Fall wird das ventrikuläre Ausstoßintervall um die Dauer verringert und eine neue Reihe von Impulsen mit der gleichen Amplitude wird durchgeführt, wobei im entgegengesetzten Fall die Amplitude der Impulse der vorherigen Reihe als Schwellenwert definiert wird.

12. Verfahren nach Anspruch 6, **dadurch gekennzeichnet, dass**, wenn der Schrittmacher in einer DDD-Betriebsart arbeitet, nach einer Reihe von Impulsen, die nicht alle zum gleichen Ergebnis führen, überprüft wird, ob die Zeitspanne AV um zumindest eine Dauer von ungefähr 32 ms größer ist als ein Wert vorzugsweise gleich 62 ms: in diesem Fall,wird die Zeitspanne AV um die Dauer verringert und eine neue Reihe von Impulsen mit der gleichen Amplitude wird durchgeführt, wobei im entgegengesetzten Fall die Amplitude der Impulse der vorherigen Reihe als Schwellenwert definiert wird.

13. Verfahren nach Anspruch 1, **dadurch gekennzeichnet, dass** die Stimulationsenergie dadurch bestimmt wird, dass der doppelte Erfassungsschwellenwert als Amplitude der Stimulationsimpulse genommen wird.

14. Verfahren nach Anspruch 1, **dadurch gekennzeichnet, dass** während der Abgleichphase nach jeder Stimulation bei 2,5 V eine Gegen-Stimulation ausgelöst wird.

15. Verfahren nach Anspruch 1, **dadurch gekennzeichnet, dass** während der Schwellenwertsuchphase im Falle einer unwirksamen Stimulation eine Gegen-Stimulation ausgelöst wird.

16. Verfahren nach Anspruch 1, **dadurch gekennzeichnet, dass** außerhalb der Abgleichphase und der Schwellenwertsuchphase außer im Falle einer Stimulation mit hoher Energie die Wirksamkeit jeder Stimulation überprüft wird, wobei im Falle einer unwirksamen Stimulation die Stimulationen der folgenden Zyklen, vorzugsweise vier an der Zahl, mit hoher Energie geliefert werden.

17. Verfahren nach Anspruch 1, **dadurch gekennzeichnet, dass** die wirksamkeitsrate der Stimulationen mittels eines Zweirichtungszählers bewertet wird, auf den ein Inkrement von vorzugsweise 1 für jede wirksame Stimulation und ein Dekrement von vorzugsweise 4 für jede unwirksame Stimulation angewendet wird, und wenn der Zähler auf 0 abnimmt, wird die Wirksamkeitsrate der Stimulationen als ungenügend definiert und eine Abgleichphase ausgelöst.

18. Verfahren nach Anspruch 1, **dadurch gekennzeichnet, dass** während der Abgleichphase vorzugsweise höchstens dreimal die Kohärenz der Stimulationsreihen überprüft wird.

19. Verfahren nach einem der Ansprüche 11 oder 12, **dadurch gekennzeichnet, dass** während der Schwellenwertsuchphase die Verringerung der Herzperiode vorzugsweise höchstens dreimal durchgeführt wird, um die Wirksamkeit der Stimulationen zu bestimmen.

20. Verfahren nach Anspruch 1, **dadurch gekennzeichnet, dass** zwischen einer chronische Situation und einer akuten Situation entsprechend einer Zeitdauer von ungefähr einem Monat ab der Implantation des Schrittmachers unterschieden wird.

21. verfahren nach Anspruch 20, **dadurch gekennzeichnet, dass** die Periodizität der Abgleichphasen bei einer chronischen Situation 24 Stunden und bei einer akuten Situation 6 Stunden beträgt.

22. Verfahren nach Anspruch 20, **dadurch gekennzeichnet, dass** die Periodizität der Schwellenwertsuchphasen bei einer chronischen Situation 6 Stunden und bei einer akuten Situation 1,5 Stunden beträgt.

23. Verfahren nach Anspruch 1, **dadurch gekennzeichnet, dass** die Schwellenwertsuchphase nicht ausgelöst wird, wenn die Abgleichphase einen Wert größer als 2,5 V als Schwellenwert definiert.

24. Verfahren nach Anspruch 1 zur Regelung der Stimulationsenergie eines Stimulationsimpulses mit einer Amplitude und einer Breite zum Stimulieren, die von einem Herzschrittmacher geliefert werden, wobei das Verfahren **gekennzeichnet ist durch** Schritte zum
- Überwachen von intrakavitären Signalen als Reaktion auf den **durch** den Schrittmacher gelieferten Stimulationsimpuls und Lieferung des für die Herzreaktion repräsentativen Reaktionswerts;
- Definieren des Erfassungsschwellenwerts, der einer minimalen Stimulationsimpulsenergie entspricht, die einen gewünschten Herzreaktionswert erzeugt;
- Regeln der dem definierten Wert des Erfassungsschwellenwerts entsprechenden Stimulationsimpulsenergie;
- Definieren, dass ein gelieferter Stimulationsimpuls wirksam ist, wenn der von der Herzreaktion gelieferte Wert größer als der definierte Erfassungsschwellenwert ist, und unwirksam ist, wenn der von der Herzreaktion gelieferte Wert kleiner als der definierte Erfassungsschwellenwert ist, und
- Erzeugen einer ausgewählten Anzahl von Hochenergie-Stimulationsimpulsen als Reaktion auf einen als unwirksam definierten Stimulationsimpuls.

25. Verfahren nach Anspruch 24, **dadurch gekennzeichnet, dass** ferner wirksame und unwirksame Stimulationsimpulse gezählt werden, die Zählung jedes Mal inkrementiert wird, wenn der gelieferte Stimulationsimpuls wirksam ist, die Zählung jedes Mal dekrementiert wird, wenn der gelieferte Stimulationsimpuls unwirksam ist, und ein neuer Erfassungsschwellenwert als Reaktion auf eine Verringerung der zählung bis auf einen vorbestimmten Zählwert definiert wird.

26. Verfahren nach Anspruch 25, **dadurch gekennzeichnet, dass** ein Inkrement von Eins auf die Zählung für jeden als wirksam definierten Stimulationsimpuls bis hin zu einem maximalen Zählwert angewendet wird und ein Dekrement von Vier für jeden als unwirksam definierten Stimulationsimpuls angewendet wird, und dadurch, dass der vorbestimmte Zählwert Null ist.

27. Verfahren nach Anspruch 24, **dadurch gekennzeichnet, dass** die Definition des Erfassungsschwellenwerts ferner folgende Schritte aufweist:
- Erzeugen einer Vielzahl von Abgleichstimulationsimpulsen mit bestimmter Amplitude und Breite;
- Bestimmen, ob die Abgleichstimulationsimpulse wirksam sind oder nicht;
- Einstellen der Amplitude der Abgleichstimulationsimpulse zur Identifizierung der minimalen Impulsamplitude für die gegebene Breite, die einem wirksamen Stimulationsimpuls entspricht, und
- Definieren des Erfassungsschwellenwerts ausgehend von der identifizierten minimalen Amplitude der Abgleichstimulationsimpulse.

28. Verfahren nach Anspruch 24, **dadurch gekennzeichnet, dass** die Definition des Erfassungsschwellenwerts ferner folgende Schritte umfasst:
i. Erzeugen einer Reihe einer ersten Anzahl von Abgleichstimulationsimpulsen mit einer bestimmten Breite und Amplitude;
ii. Bestimmen, ob jeder Impuls der ersten Anzahl von Impulsen der erzeugten Reihe wirksam ist, und,
iii. als Reaktion auf die Tatsache, dass jeder Impuls der ersten Anzahl von Impulsen der Reihe wirksam ist,
a) Verringern der Amplitude des Abgleichstimulationsimpulses um eine vorbestimmte Größe und Erzeugen einer weiteren Reihe der ersten Anzahl von Abgleichstimulationsimpulsen mit der verringerten Impulsamplitude;
b) Bestimmen, ob jeder Impuls der ersten Anzahl von Impulsen der erzeugten Reihe wirksam ist;
c) als Reaktion auf die Tatsache, dass jeder Impuls der ersten Anzahl von Impulsen unwirksam ist, Definieren des Erfassungsschwellenwerts als Amplitude der Abgleichstimulationsimpulse der vorherigen Reihe, und,
d) als Reaktion auf die Tatsache, dass ein Impuls der ersten Anzahl von Impulsen wirksam ist, Wiederholen des Schritts iii.

29. Verfahren nach Anspruch 28, **dadurch gekennzeichnet, dass** die erste Anzahl von Impulsen Vier ist und dass jeder Impuls die gleiche Breite und die gleiche Amplitude aufweist.

30. Verfahren nach Anspruch 28, **dadurch gekennzeichnet, dass** die Impulsbreite zwischen 0,5 ms und 1,0 ms gewählt wird und die bestimmte Größe zwischen 0,25 und 0,5 Volt gewählt wird.

31. Verfahren nach Anspruch 24, **dadurch gekennzeichnet, dass** die Definition des Erfassungsschwellenwerts einen Schritt aufweist zum Halten des definierten Erfassungsschwellenwerts auf einem Wert größer oder gleich einem minimalen Erfassungsschwellenwert, der einer minimalen Amplitude und Impulsbreite entspricht.

32. Verfahren nach Anspruch 28, bei dem der Schrittmacher in einer VVI-Betriebsart mit einem ventrikulären Ausstoßintervall und einem Herzzyklus arbeitet, **dadurch gekennzeichnet, dass** der Schritt ii. ferner Unterschritte umfasst: zum Bestimmen, ob die Reihe von Stimulationsimpulsen zumindest einen wirksamen Abgleichstimulationsimpuls und einen unwirksamen Abgleichstimulationsimpuls aufweist, und, als Reaktion darauf, zum Bestimmen, ob die Zeitdauer des analysierten Herzzyklus um zumindest eine erste Dauer größer ist als eine bestimmte minimale Zeitdauer, wobei in diesem Fall das ventrikuläre Ausstoßintervall um die erste Dauer verringert wird und die Schritte i-iii mit Abgleichstimulationsimpulsen mit der gleichen Amplitude wie die vorherige Reihe wiederholt werden und wobei im entgegengesetzten Fall der Erfassungsschwellenwert als Amplitude der Impulse der vorherigen Reihe definiert wird.

33. Verfahren nach Anspruch 28, bei dem der Schrittmacher in einer DDD-Betriebsart mit einer Zeitspanne AV arbeitet, **dadurch gekennzeichnet, dass** der schritt ii ferner Unterschritte aufweist: zum Bestimmen, ob die erste Reihe von Abgleichstimulationsimpulsen zumindest einen wirksamen Impuls und zumindest einen unwirksamen Impuls aufweist, und, als Reaktion darauf, zum Bestimmen, ob die Zeitspanne AV um zumindest eine erste Größe größer als ein vorbestimmter Wert ist, wobei in diesem Fall die Zeitspanne AV um die erste Größe verringert wird und die Schritte i-iii mit den Abgleichstimulationsimpulsen mit der gleichen Impulsamplitude wie im vorhergehenden Zyklus wiederholt werden und wobei im entgegengesetzten Fall der Erfassungsschwellenwert als die Amplitude der Impulse der vorherigen Reihe definiert wird.

34. Verfahren nach Anspruch 24, **dadurch gekennzeichnet, dass** der Schritt zum Definieren des Erfassungsschwellenwerts ferner die Bestimmung eines neuen Erfassungsschwellenwerts gemäß einer Periodizität von ungefähr 24 Stunden bei einer chronischen Situation und von ungefähr 6 Stunden bei einer akuten Situation umfasst.

35. Verfahren nach Anspruch 24, **dadurch gekennzeichnet, dass** der Schritt zum Definieren des Erfassungsschwellenwerts ferner Unterschritte umfasst zum:
- Definieren eines Erfassungsparameters, der entsprechend der Stimulationsenergie variiert,
- Vorsehen einer Abgleichphase zur Bestimmung von repräsentativen Merkmalen des Erfassungsparameters entsprechend der analysierten Herzreaktion bei einem oder mehreren Abgleichstimulationsimpulsen,
- Definieren eines Referenzwertes zur Bestimmung der Wirksamkeit von Abgleichstimulationsimpulsen als Reaktion auf bestimmte repräsentative Merkmale des Erfassungsparameters, und
- Vorsehen einer Schwellenwertsuchphase zur Bestimmung des Erfassungsschwellenwerts in Bezug auf den definierten Referenzwert.

36. Gerät zur Regelung der Stimulationsenergie in einem Herzschrittmacher, mit:
- einem Impulsgenerator zur Erzeugung von Impulsen mit einer bestimmten Stimulationsenergie,
- einer Überwachungseinrichtung zur Erfassung der Herzreaktion auf die Stimulationsenergie eines gelieferten Impulses,
- Einrichtungen zur Steuerung der gelieferten Stimulationsenergie in Bezug auf einen bestimmten Erfassungsschwellenwert,
**dadurch gekennzeichnet, dass** das Gerät Einrichtungen zur Definition des Erfassungsschwellenwerts gemäß einem Abgleichsverfahren aufweist, bei dem repräsentative Merkmale eines Erfassungsparameters, der von einer maximalen negativen Amplitude des Herzsignals gebildet wird, das nach der Stimulation in einer bestimmten Zeitspanne erhalten wird, entsprechend der analysierten Stimulationsenergie als Reaktion auf einen oder mehrere Impulse bestimmt werden, wobei der Erfassungsparameter entsprechend der Stimulationsenergie variiert, und bei dem ein Referenzwert zur Bestimmung der Wirksamkeit eines Impulses als Reaktion auf bestimmte repräsentative Merkmale des Erfassungsparameters definiert wird, und gemäß einem Schwellenwertsuchverfahren aufweist, bei dem der Erfassungsschwellenwert auf der Grundlage des definierten Referenzwerts bestimmt wird.

37. Gerät nach Anspruch 36 zur Regelung der Stimulationsenergie von Stimulationsimpulsen, die von einem Herzschrittmacher geliefert werden, **dadurch gekennzeichnet, dass**
- der Generator Stimulationsimpulse mit einer Amplitude und einer Breite erzeugt, die definiert werden können und einer Stimulationsenergie entsprechen;
- die Überwachungseinrichtung die intrakavitäre Reaktion auf die von dem Schrittmacher gelieferten Stimulationsimpulse erfasst und einen für die Herzreaktion repräsentativen Reaktionswert liefert;
und dadurch, dass es aufweist:
- Einrichtungen zur Definition des Erfassungsschwellenwerts, so dass er der minimalen, einen bestimmten Herzreaktionswert erzeugenden Stimulationsimpulsenergie entspricht, und
- Einrichtungen zur Bestimmung, dass der gelieferte Stimulationsimpuls wirksam ist, wenn der Wert der Herzreaktion größer als der definierte Erfassungsschwellenwert ist, und unwirksam ist, wenn der gelieferte Wert der Herzreaktion kleiner als der definierte Erfassungsschwellenwert ist, so dass die Steuerungseinrichtungen durch den Generator eine bestimmte Anzahl von Stimulationsimpulsen mit einer bestimmten Amplitude und Breite erzeugen lassen, die einem Hochenergie-Impuls als Reaktion auf einen als unwirksam definierten Stimulationsimpuls entsprechen.

38. Gerät nach Anspruch 37, **dadurch gekennzeichnet, dass** es ferner aufweist: einen Zähler, um wirksame und unwirksame Stimulationsimpulse zu zählen, wobei der Zähler inkrementiert wird, wenn der gelieferte Stimulationsimpuls wirksam ist, und dekrementiert wird, wenn der gelieferte Stimulationsimpuls unwirksam ist, so dass die Definitionseinrichtungen einen neuen Erfassungsschwellenwert definieren, wenn der Zähler auf einen vorbestimmten Zählwert abfällt.

39. Gerät nach Anspruch 38, **dadurch gekennzeichnet, dass** der Zähler für jeden als wirksam bestimmten Stimulationsimpuls um Eins bis auf einen maximalen Zählwert inkrementiert wird, und für jeden als unwirksam bestimmten Stimulationsimpuls um Vier dekrementiert wird, und dadurch, dass der vorbestimmte Zählwert Null ist.

40. Gerät nach Anspruch 37, **dadurch gekennzeichnet, dass** die Definitionseinrichtungen ferner aufweisen: Einrichtungen, die auf den Generator einwirken, um eine Vielzahl von Abgleichstimulationsimpulsen mit einer bestimmten Amplitude und Breite zu erzeugen; einen Komparator zur Bestimmung, ob die Abgleichstimulationsimpulse wirksam sind oder nicht, so dass die auf den Generator einwirkenden Einrichtungen die Amplitude der Abgleichstimulationsimpulse einstellen, um die minimale Impulsamplitude zu identifizieren, die einem wirksamen Stimulationsimpuls für eine gegebene Impulsbreite entspricht, und dass die Definitionseinrichtungen den Erfassungsschwellenwert ausgehend von der identifizierten minimalen Amplitude der Abgleichstimulationsimpulse definieren.

41. Gerät nach Anspruch 37, **dadurch gekennzeichnet, dass** die Definitionseinrichtungen ferner aufweisen:
- auf den Generator einwirkende Einrichtungen zur Erzeugung einer Reihe einer ersten Anzahl von Abgleichs-Stimulationsimpulsen mit einer bestimmten Breite und Amplitude;
- Einrichtungen zur Bestimmung, ob jeder der ersten Anzahl von Impulsen der erzeugten Reihe wirksam ist, dergestalt, dass als Reaktion auf die Tatsache, dass jeder Impuls der ersten Anzahl wirksam ist, die einwirkenden Einrichtungen in dem Generator eine Reihe von Abgleichstimulationsimpulsen mit einer um eine vorbestimmte Größe in Bezug auf die Impulsamplitude der vorherigen Reihe reduzierten Amplitude erzeugen lassen, und die Bestimmungseinrichtungen bestimman, ob jeder der ersten Anzahl von Impulsen der erzeugten Reihe wirksam ist, und dergestalt, dass als Reaktion auf die Tatsache, dass jeder der ersten Anzahl von Impulsen unwirksam ist, die Definitionseinrichtungen den Erfassungsschwellenwert als die Amplitude der Abgleichstimulationsimpulse der vorherigen Impulsreihe definieren, und dergestalt, dass die Einrichtungen, die als Reaktion auf die Tatsache, dass einer der ersten Anzahl von Impulsen wirksam ist, tätig werden, bewirken, dass der Generator und die Bestimmungseinrichtungen weiterhin aufeinanderfolgende Impulsreihen mit einer immer weiter verringerten Amplitude erzeugen, bis die Definitionseinrichtungen einen Erfassungsschwellenwert definieren.

42. Gerät nach Anspruch 41, **dadurch gekennzeichnet, dass** die erste Anzahl von Impulsen vier ist und dass jeder Impuls derselben Reihe die gleiche Breite und Amplitude aufweist.

43. Gerät nach Anspruch 42, **dadurch gekennzeichnet, dass** die Impulsbreite zwischen 0,5 ms und 1,0 ms und die vorbestimmte Größe zwischen 0,25 und 0,5 V liegt.

44. Gerät nach Anspruch 37, **dadurch gekennzeichnet, dass** die Definitionseinrichtungen ferner einen Komparator aufweisen, um den von der Herzreaktion gelieferten Wert mit einem bestimmten minimalen Erfassungsschwellenwert zu vergleichen und um den Erfassungsschwellenwert an den bestimmten minimalen Wert zu liefern, wenn der von der Herzreaktion gelieferte Wert nicht größer als der bestimmte Minimalwert ist.

45. Gerät nach Anspruch 41, bei dem der Schrittmacher in einer VVI-Betriebsart mit einem ventrikulären Ausstoßintervall arbeitet, **dadurch gekennzeichnet, dass** es ferner aufweist:
- erste Einrichtungen zur Bestimmung, ob die Abgleichstimulationsimpulse der Reihe zumindest einen wirksamen und einen unwirksamen Abgleichstimulationsimpuls erzeugen,
- zweite Einrichtungen zur Bestimmung, ob die Zeitdauer eines analysierten Herzzyklus in zumindest einer ersten Dauer größer ist als eine bestimmte minimale Zeitdauer, und
- Einrichtungen zur Verringerung des ventrikulären Ausstoßintervalls um eine erste Dauer als Reaktion auf Abgleichstimulationsimpulse, die zumindest einen wirksamen Stimulationsimpuls und einen unwirksamen Impuls erzeugen, wenn der gemessene Herzzyklus um mindestens eine erste Dauer größer als die bestimmte minimale Zeitdauer ist, dergestalt, dass als Reaktion auf eine Verringerung des ventrikulären Ausstoßintervalls die auf den Generator einwirkenden Einrichtungen in dem Generator eine Reihe von Abgleichstimulationsimpulsen mit einer zu der der vorherigen Reihe identischen Impulsamplitude erzeugen lassen und als Reaktion auf das Fehlen einer Verringerung des ventrikulären Ausstoßintervalls die Definitionseinrichtungen den Schwellenwert als gleich zu der Amplitude der Abgleichstimulationsimpulse der vorherigen Reihe definieren.

46. Gerät nach Anspruch 41, bei der der Schrittmacher in einer DDD-Betriebsart mit einer Zeitspanne AV arbeitet, **dadurch gekennzeichnet, dass** es ferner aufweist:
- erste Einrichtungen zur Bestimmung, ob die Reihe zumindest einen wirksamen und einen unwirksamen Abgleichstimulationsimpuls erzeugt,
- Einrichtungen zur Bestimmung, ob die Zeitspanne AV größer als eine erste Größe mit einem bestimmten Wert ist, und
- Einrichtungen zur Verringerung der Zeitspanne AV der ersten Größe in dem Fall, dass die Zeitspanne AV um die erste Größe größer als der bestimmte Wert ist, dergestalt, dass als Reaktion auf eine Verringerung der Zeitspanne AV die auf den Generator einwirkenden Einrichtungen in dem Generator eine Reihe von Abgleichstimulationsimpulsen mit einer zu der der vorherigen Reihe identischen Amplitude erzeugen lassen und als Reaktion auf das Fehlen einer Verringerung der Zeitspanne AV die Definitionseinrichtungen den Schwellenwert als gleich zu der Amplitude der Abgleichstimulationsimpulse der vorherigen Reihe definieren.

47. Gerät nach Anspruch 37, **dadurch gekennzeichnet, dass** die Definitionseinrichtungen periodisch einen neuen Erfassungsschwellenwert gemäß einer Periodizität von ungefähr 24 Stunden bei einer chronischen Situation und von ungefähr 6 Stunden bei einer akuten Situation definieren.

48. Gerät nach Anspruch 37, **dadurch gekennzeichnet, dass** die Definitionseinrichtungen ferner Einrichtungen aufweisen, um den Erfassungsschwellenwert gemäß einem Abgleichverfahren zu bestimmen, dergestalt dass repräsentative Merkmale eines Erfassungsparameters entsprechend der mit einem oder mehreren Abgleichstimulationsimpulsen analysierten Herzreaktion bestimmt werden, wobei der Erfassungsparameter entsprechend der Stimulationsenergie variiert, und dergestalt, dass ein Referenzwert definiert ist, um die Wirksamkeit der Stimulationsimpulse als Reaktion auf die bestimmten repräsentativen Merkmale des Erfassungsparameters zu bestimmen, und gemäß einem Schwellensuchverfahren zu bestimmen, dergestalt, dass der Erfassungsschwellenwert auf der Grundlage des bestimmten Referenzwerts bestimmt wird.

## Claims

1. A method of regulating, by means of software, the pacing energy in a cardiac pacemaker by measurement of a capture parameter, comprising the following steps:
- a capture parameter is defined constituted by the maximum negative amplitude of the cardiac signal obtained after pacing, within a determined period, as a function of the pacing energy;
- the representative characteristics of the capture parameter as a function of the pacing energy are determined during a calibration phase;
- a reference value for determining the effectiveness of the pacing operations is defined from these characteristics;
- the capture threshold value is determined from the reference value during a threshold search phase;
- and the pacing energy is determined as a function of the value of the capture threshold.

2. A method according to Claim 1, **characterised in that** the determined period starts after the blanking and ends approximately 64 ms after the pacing operation.

3. A method according to Claim 1, **characterised in that** in the event that the capture parameter varies linearly as a function of the pacing energy, the characteristics of the straight line representative of the capture parameter are the slope (a) and the ordinate at the origin (b).

4. A method according to Claim 3, **characterised in that** the reference value for determining the effectiveness of the pacing operations is a fraction of the ordinate at the origin (b) preferably equal to 3/4.

5. A method according to Claim 1, **characterised in that** at the start of the calibration phase or of the threshold search phase, if possible the period of the cardiac cycle is reduced so as to avoid the phenomenon of fusion.

6. A method according to Claim 1, **characterised in that** for determining the value of the capture threshold, successive series of stimulus pulses are produced by decreasing the amplitude of the pulses between the series.

7. A method according to Claim 6, **characterised in that** each series of pulses is preferably constituted of four pulses of the same amplitude.

8. A method according to Claim 6, **characterised in that**, after each series of stimulus pulses which have all been effective, the amplitude is reduced by a determined quantity, preferably 0.25 volts.

9. A method according to Claim 8, **characterised in that**, when the amplitude of the stimulus pulses is of the order of 1.25 volts, this value is retained as threshold value.

10. A method according to Claim 6, **characterised in that**, after a series of stimulus pulses which are all ineffective, the amplitude of the pulses of the previous series is defined as threshold value.

11. A method according to Claim 6, **characterised in that**, if the pacemaker is operating in VVI mode, after a series of stimulus pulses which do not all yield the same result, it is determined whether the period of the cardiac cycle is greater by at least one duration of approximately 32 ms than the minimum permitted period: **in that** case, the ventricular escape interval is reduced by said duration and a new series of stimulus pulses with the same amplitude is carried out, and in the opposite case the amplitude of the stimulus pulses of the previous series is defined as threshold value.

12. A method according to Claim 6, **characterised in that**, if the pacemaker is operating in DDD mode, after a series of stimulus pulses which do not all yield the same result, it is determined whether the AV delay is greater by at least one duration of approximately 32 ms than a value preferably equal to 62 ms: **in that** case, the AV delay is reduced by said duration, and a new series of stimulus pulses with the same amplitude is carried out, and in the contrary case the amplitude of the stimulus pulses of the previous series is defined as threshold value.

13. A method according to Claim 1, **characterised in that** the pacing energy is determined by taking as the amplitude of the stimulus pulses twice the value of the capture threshold.

14. A method according to Claim 1, **characterised in that**, during the calibration phase, after each pacing operation at 2.5 volts, a counter-pacing operation is triggered.

15. A method according to Claim 1, **characterised in that**, during the threshold search phase, in the event of an ineffective pacing operation, a counter-pacing operation is triggered.

16. A method according to Claim 1, **characterised in that**, outside the calibration and threshold search phases, except in the event of a high-energy pacing operation, the effectiveness of each pacing operation is determined, and in the event of a pacing operation which is ineffective, the pacing operations of the following cycles, preferably four in number, are delivered with high energy.

17. A method according to Claim 1, **characterised in that** the effectiveness of the pacing operations is evaluated by means of a reversible counter to which there is applied an increment of 1, preferably, for each effective pacing operation, and a decrement of 4, preferably, for each ineffective pacing operation, and when the counter returns to 0, the effectiveness of the pacing operations is defined as insufficient, and a calibration phase is triggered.

18. A method according to Claim 1, **characterised in that** during the calibration phase the coherence of the series of pacing operations is preferably checked at most three times.

19. A method according to one of Claims 11 or 12, **characterised in that**, during the threshold search phase, preferably the cardiac period is reduced at most three times to determine the effectiveness of the pacing operations.

20. A method according to Claim 1, **characterised in that** a chronic situation and an acute situation are distinguished corresponding to a period of approximately one month from implantation of the pacemaker.

21. A method according to Claim 20, **characterised in that** the periodicity of the calibration phases is 24 hours in a chronic situation and 6 hours in an acute situation.

22. A method according to Claim 20, **characterised in that** the periodicity of the threshold search phases is 6 hours in a chronic situation and 1 hour 30 min. in an acute situation.

23. A method according to Claim 1, **characterised in that**, when the calibration phase defines a value greater than 2.5 volts for the threshold, the threshold search phase is not triggered.

24. A method according to Claim 1 for regulating the pacing energy of a stimulus pulse having an amplitude and a width for pacing, delivered by a cardiac pacemaker, **characterised by** the following steps:
- monitoring the endocardial signals in response to said stimulus pulse delivered by the pacemaker and providing a response value representative of the endocardial response;
- defining the capture threshold corresponding to a minimum stimulus pulse energy producing a desired value of endocardial response;
- regulating the stimulus pulse energy corresponding to the defined value of the capture threshold;
- defining that a delivered stimulus pulse is effective if the endocardial response value provided is greater than the defined capture threshold, and ineffective if the endocardial response value provided is less than the defined capture threshold, and
- producing a selected number of high-energy stimulus pulses in response to a stimulus pulse which is defined as ineffective.

25. A method according to Claim 24, **characterised in that** furthermore: a count of the effective and ineffective stimulus pulses is kept, the count is incremented each time the stimulus pulse provided is effective; the count is decremented each time the stimulus pulse provided is ineffective; and a new capture threshold is defined in response to a reduction in the count to a predetermined count value.

26. A method according to Claim 25, **characterised in that** an increment of one is applied to the count for each stimulus pulse which is defined as effective up to a maximum value of the count and a decrement of four is applied for each stimulus pulse which is defined as ineffective, and **in that** said predetermined count value is zero.

27. A method according to Claim 24, **characterised in that** the definition of the capture threshold furthermore comprises the following steps:
- producing a plurality of calibrating stimulus pulses having a determined amplitude and width;
- determining whether or not the calibrating stimulus pulses are effective;
- adjusting the amplitude of the calibrating stimulus pulses to identify the minimum pulse amplitude for the given width corresponding to an effective stimulus pulse, and
- defining the capture threshold from the identified minimum amplitude of the calibrating stimulus pulses.

28. A method according to Claim 24, **characterised in that** the definition of the capture threshold furthermore comprises the following steps:
ID 2057 0 1. producing a series of a first number of calibrating stimulus pulses having a determined amplitude and width;
ii. determining whether each pulse of the first number of pulses of the series produced is effective, and
iii. in response to the fact that each pulse of the first number of pulses of the series is effective,
a) reducing the amplitude of the calibrating stimulus pulse by a predetermined quantity and producing another series of the first number of calibrating stimulus pulses at the reduced pulse amplitude;
b) determining whether each pulse of the first number of pulses of the series produced is effective;
c) in response to the fact that each pulse of the first number of pulses is ineffective, defining the capture threshold as being the amplitude of the calibrating stimulus pulses of the previous series, and,
d) in response to the fact that a pulse of the first number of pulses of the series is effective, recommencing step iii.

29. A method according to Claim 28, **characterised in that** the first number of pulses is four, and **in that** each pulse has the same width and the same amplitude.

30. A method according to Claim 28, **characterised in that** the pulse width is selected between 0.5 ms and 1.0 ms and the quantity determined is selected between 0.25 and 0.5 volts.

31. A method according to Claim 24, **characterised in that** the definition of the capture threshold comprises: a step consisting of maintaining the defined capture threshold at a value equal to or greater than a minimum capture threshold corresponding to a minimum amplitude and pulse width.

32. A method according to Claim 28, in which the pacemaker operates in VVI mode with a ventricular escape interval and a cardiac cycle, **characterised in that** step ii. furthermore comprises: determining whether the series of stimulus pulses comprises at least one effective calibrating stimulus pulse and one ineffective calibrating stimulus pulse, and, in response, determining whether the period of the cardiac cycle analysed is greater by at least one first duration than a determined minimum period , and, in this case, reducing the ventricular escape interval by the first duration and recommencing steps i-iii with calibrating stimulus pulses with the same amplitude as the previous series and, in the opposite case, defining the capture threshold value as being the amplitude of the pulses of the previous series.

33. A method according to Claim 28, in which the pacemaker operates in DDD mode with an AV delay, **characterised in that** step ii. furthermore comprises: determining whether the first series of calibrating stimulus pulses comprises at least one effective pulse and at least one ineffective pulse, and, in response, determining whether the AV delay is greater by at least one first quantity than a predetermined value and, **in that** case, reducing the AV delay by the first quantity and recommencing steps i-iii with calibrating stimulus pulses at the same pulse amplitude as in the previous cycle, and, in the opposite case, defining the capture threshold value as being the amplitude of the pulses of the previous series.

34. A method according to Claim 24, **characterised in that** the step of defining the capture threshold furthermore comprises: the determination of a new capture threshold at a periodicity of approximately 24 hours in a chronic situation and approximately 6 hours in an acute situation.

35. A method according to Claim 24, **characterised in that** the step of defining the capture threshold furthermore comprises:
- defining a capture parameter which varies as a function of the pacing energy;
- providing a calibration phase for determining representative characteristics of the capture parameter as a function of the analysed endocardial response to one or more calibrating stimulus pulses;
- defining a reference value for determining the effectiveness of calibrating stimulus pulses in response to the determined representative characteristics of the capture parameter; and
- providing a threshold search phase for determining the value of the capture threshold relative to the defined reference value.

36. A device for regulating the pacing energy in a cardiac pacemaker, comprising:
- a pulse generator for producing pulses having a determined pacing energy,
- a monitor for sensing the endocardial response to the pacing energy of a delivered pulse,
- means for controlling the pacing energy provided relative to a determined capture threshold value,
**characterised in that** it comprises means for defining the capture threshold according to a calibration process in which representative characteristics of a capture parameter constituted by the maximum negative amplitude of the cardiac signal obtained after pacing within a determined period are determined as a function of the pacing energy analysed in response to one or more pulses, the capture parameter varying as a function of the pacing energy, and in which a reference value is defined to determine the effectiveness of a pulse in response to the determined representative characteristics of the capture parameter, and according to a threshold search process in which the value of the capture threshold is determined on the basis of the defined reference value.

37. A device according to Claim 36 for regulating the pacing energy of stimulus pulses delivered by a cardiac pacemaker, **characterised in that**:
- the generator produces stimulus pulses having an amplitude and a width which can be defined and correspond to a pacing energy;
- the monitor senses the endocardial response to the stimulus pulses delivered by the pacemaker and provides a response value representative of the endocardial response;
and **in that** it comprises:
- means for defining the capture threshold to correspond to the minimum stimulus pulse energy producing a determined value of endocardial response, and
- means for determining that the stimulus pulse delivered is effective when the value of the endocardial response is greater than the defined capture threshold and ineffective when the provided value of the endocardial response is less than the defined capture threshold, such that the control means cause the generator to produce a determined number of stimulus pulses having a determined amplitude and width corresponding to a high-energy pulse in response to a stimulus pulse which is defined as ineffective.

38. A device according to Claim 37, **characterised in that** it furthermore comprises: a counter for keeping a count of the effective and ineffective stimulus pulses, the counter being incremented when the stimulus pulse delivered is effective and decremented when the stimulus pulse delivered is ineffective, such that the definition means define a new capture threshold when the counter drops to a predetermined count value.

39. A device according to Claim 38, **characterised in that** the counter is incremented by one for each stimulus pulse which is determined as effective up to a maximum count value, and decremented by four for each stimulus pulse which is determined as ineffective, and **in that** the predetermined count value is zero.

40. A device according to Claim 37, **characterised in that** the definition means furthermore comprise: means acting on the generator to produce a plurality of calibrating stimulus pulses having a determined amplitude and width; a comparator for determining whether or not the calibrating stimulus pulses are effective: such that the means acting on the generator adjust the amplitude of the calibrating stimulus pulses to identify the minimum pulse amplitude corresponding to an effective stimulus pulse for a given pulse width; and that the definition means define the capture threshold from the identified minimum amplitude of the calibrating stimulus pulses.

41. A device according to Claim 37, **characterised in that** the definition means furthermore comprise:
- means acting on the generator to produce a series of a first number of calibrating stimulus pulses having a determined amplitude and width;
- means for determining whether each of the first number of pulses of the series generated is effective, such that, in response to the fact that each pulse of the first number is effective, the acting means make the generator produce a series of calibrating stimulus pulses having an amplitude reduced by a predetermined quantity relative to the pulse amplitude of the previous series, and the determination means determine whether each of the first number of pulses of the series generated is effective, and such that, in response to the fact that each of the first number of pulses is ineffective, the definition means define the capture threshold as being the amplitude of the calibrating stimulus pulses of the previous series of pulses; and such that the acting means, in response to the fact that one of the first number of pulses is effective, cause the generator and the determination means to continue to produce successive series of pulses of increasingly reduced amplitude until the definition means define a capture threshold.

42. A device according to Claim 41, **characterised in that** the first number of pulses is four, and **in that** each pulse of the same series has the same width and the same amplitude.

43. A device according to Claim 42, **characterised in that** the pulse width is selected between 0.5 ms and 1.0 ms and the predetermined quantity is selected between 0.25 and 0.5 volts.

44. A device according to Claim 37, **characterised in that** the definition means furthermore comprise a comparator for comparing the provided value of the endocardial response with a determined minimum capture threshold, and for providing the capture threshold at the minimum value determined if the provided value of the endocardial response is not greater than the determined minimum value.

45. A device according to Claim 41, in which the pacemaker operates in VVI mode with a ventricular escape interval, **characterised in that** it furthermore comprises:
- first means for determining whether the calibrating stimulus pulses of the series produce at least one effective calibrating stimulus pulse and one ineffective calibrating stimulus pulse;
- second means for determining whether the period of an analysed cardiac cycle is greater by at least one first duration than a determined minimum period; and
- means for reducing the ventricular escape interval by a first duration in response to calibrating stimulus pulses producing at least one effective stimulus pulse and one ineffective pulse when the measured cardiac cycle is greater by at least one first duration than the determined minimum period, such that, in response to a reduction in the ventricular escape interval, the means acting on the generator cause the generator to produce a series of calibrating stimulus pulses of a pulse amplitude identical to that of the previous series, and, in response to the absence of a reduction in the ventricular escape interval, the definition means define the threshold value as equal to the amplitude of the calibrating stimulus pulses of the previous series.

46. A device according to Claim 41, in which the pacemaker operates in DDD mode with an AV delay, **characterised in that** it furthermore comprises:
- first means for determining whether the series produces at least one effective calibrating stimulus pulse and one ineffective calibrating stimulus pulse;
- means for determining whether the AV delay is greater by a first quantity than a determined value; and
- means for reducing the AV delay by the first quantity in the event that the AV delay is greater by the first quantity than the determined value, such that, in response to a reduction in the AV delay, the means acting on the generator cause the generator to produce a series of calibrating stimulus pulses of an amplitude identical to that of the previous series, and, in response to the absence of a reduction in the AV delay, the definition means define the threshold value as equal to the amplitude of the calibrating stimulus pulses of the previous series.

47. A device according to Claim 37, **characterised in that** the definition means periodically define a new capture threshold at a periodicity of approximately 24 hours in a chronic situation and approximately 6 hours in an acute situation.

48. A device according to Claim 37, **characterised in that** the definition means furthermore comprise means for determining the capture threshold according to a calibration process, such that representative characteristics of a capture parameter are determined as a function of the analysed endocardial response to one or more calibrating stimulus pulses, the capture parameter varying as a function of the pacing energy, and such that a reference value is defined to determine the effectiveness of the stimulus pulses in response to the determined representative characteristics of the capture parameter; and according to a threshold search process, such that the value of the capture threshold is determined on the basis of the determined reference value.
